# EUROPÄISCHE PATENTSCHRIFT

(11) **EP 1 846 569 B1**
(45) Veröffentlichungstag und Bekanntmachung des Hinweises auf die Patenterteilung: **24.02.2010**
(21) Anmeldenummer: 06704549.2
(22) Anmeldetag: 20.01.2006
(51) Int. Cl.: C12Q 1/06, C12Q 1/04

(54) **VERFAHREN ZUR BESTIMMUNG VON KEIMEN**
METHOD FOR IDENTIFYING GERMS
PROCEDE POUR DETECTER DES GERMES

(30) Priorität: 10.02.2005 DE 102005006237
(43) Veröffentlichungstag der Anmeldung: 24.10.2007
(73) Patentinhaber: Henkel AG & Co. KGaA, 40589 Düsseldorf (DE)
(72) Erfinder: FRANK, Michael, 40593 Düsseldorf (DE); SIEPMANN, Friedhelm, 45136 Essen (DE); THÜNCHEN, Andreas, 42289 Wuppertal (DE); STUMPE, Stefan, 40593 Düsseldorf (DE); HERRMANN, Helmut, 63454 Hanau (DE)
(86) Internationale Anmeldenummer: PCT/EP2006/000473
(87) Internationale Veröffentlichungsnummer: WO 2006/084552

(56) Entgegenhaltungen:
- EP-A- 0 713 087
- WO-A-2004/055203
- US-A- 4 444 317
- US-A1- 2001 055 776
- YU L S L ET AL: "Time-resolved fluorescence immunoassay (TRFIA) for the detection of Escherichia coli O157:H7 in apple cider" JOURNAL OF MICROBIOLOGICAL METHODS, Bd. 49, Nr. 1, März 2002 (2002-03), Seiten 63-68, XP002377863 ISSN: 0167-7012
- PERUSKI A H ET AL: "Rapid and sensitive detection of biological warfare agents using time-resolved fluorescence assays" JOURNAL OF IMMUNOLOGICAL METHODS, ELSEVIER, AMSTERDAM, NL, Bd. 263, Nr. 1-2, 1. Mai 2002 (2002-05-01), Seiten 35-41, XP004354383 ISSN: 0022-1759
- ANONYMOUS: "Time-resolved fluorescence based GTP binding assay for G-protein coupled receptors" INTERNET ARTICLE, [Online] Januar 2002 (2002-01), Seiten 1-8, XP002377864 Gefunden im Internet: URL:http://las.perkinelmer.com/content/App licationNotes/12349858-GTP-Eu.pdf> [gefunden am 2006-04-21]
- PERNTHALER ANNELIE ET AL: "Comparison of fluorescently labeled oligonucleotide and polynucleotide probes for the detection of pelagic marine bacteria and archaea" APPLIED AND ENVIRONMENTAL MICROBIOLOGY, Bd. 68, Nr. 2, Februar 2002 (2002-02), Seiten 661-667, XP002377865 ISSN: 0099-2240 in der Anmeldung erwähnt
- HIRSCHFELD T.: 'Fluorescence Background Discrimination by Prebleaching' J HISTOCHEM CYTOCHEM Bd. 27, Nr. 1, 1979, Seiten 96 - 101, XP002371466

## Beschreibung

Die vorliegende Erfindung betrifft ein Verfahren zur quantitativen und/oder qualitativen Bestimmung von Keimen.

Keime - hierunter werden erfindungsgemäß Bakterien, Hefen und Pilze verstanden - in Produkten jeglicher Art können einerseits zum Verderb führen, was einen direkten Einfluß auf die Qualität, Wirkungsweise und Leistung hat. Andererseits können bei zu hohen Keimbelastungen bzw. durch pathogene Keime Infektionen und/oder Krankheiten hervorgerufen werden. Daher ist die unmittelbare Erkennung einer Keimbelastung unerläßlich, z. B. bevor ein Endprodukt in den Markt gelangt.

Für eine Vielzahl von Substanzen, Rohstoffen und Produkten aus den unterschiedlichen Bereichen Industrie, Handwerk, Haushalt, Gesundheit, Gastronomie etc. muß die mikrobiologische Sicherheit gewährleistet sein. Dabei ist zu beachten, daß die Art und Anzahl unterschiedlicher Keime, wie z. B. Bakterien und Pilze, in engen Grenzen kontrolliert werden muß.

Die Kontrolle des hygienischen Status von Konsumgütern, wie Lebensmitteln, Kosmetika, Klebstoffen, Wasch- und Reinigungsmitteln, aber auch z. B. von Kühlschmierstoffen, die insbesondere im industriellen Bereich eingesetzt werden, ist vom Gesetzgeber geregelt. Je nach Art der Güter sind unterschiedliche Grenzwerte für die mikrobiologische Belastung definiert. Gleiches gilt für Oberflächen in Krankenhäusern (z. B. in Operationssälen), aber in zunehmendem Maße auch für Klimaanlagen, Wärmetauscher etc.

Die Nachweismethoden sind sehr vielfältig und hängen in ihrer Komplexität, dem apparativen Aufwand und der notwendigen Analysezeit meist von den nachzuweisenden Keimgehalten, insbesondere der Spezifikation der maximalen Keimzahl, und der Matrix, in der die Keime auftreten können, ab. Als Keime werden in der vorliegenden Anmeldung insbesondere Bakterien (Gram-positiv und Gram-negativ), Hefen und Pilze bezeichnet.

Quantitative mikrobiologische Analysetechniken werden zur Qualitätssicherung schon seit langer Zeit eingesetzt. Grundlage dieser Methoden ist es, einzelne Keime, die in dem zu untersuchenden Material vorkommen, so zu vermehren, daß sie als Ausstriche oder Kolonien mit bloßem Auge sichtbar werden. Standardmäßig werden hierfür Kultivierungsmethoden eingesetzt, die diese Keime entweder auf festen Nährböden oder in flüssigen Nährlösungen bzw. -medien vermehren. Die Durchführung herkömmlicher Kultivierungsverfahren zum Nachweis von Bakterien und Pilzen dauert je nach Methode und Keimart bis zu mehreren Tagen.

Das mitunter "klassischste" Verfahren zum Nachweis von Keimen besteht in der sogenannten Platten-Kultur-Methode. Dabei wird die zu analysierende Probe auf eine mit Nährmedium (z. B. Agar-Agar) beschichtete Petrischale aufgebracht und für eine bestimmte Zeit unter definierten Bedingungen kultiviert. Im Laufe von einem oder mehreren Tagen wachsen im Falle einer keimbelasteten Probe Kolonien auf dem Nährmedium an, die mit dem bloßen Auge detektiert und ausgezählt werden können. Auf diese relativ einfache, aber langsame Art gelingt der Keimnachweis, vorausgesetzt, daß für die nachzuweisenden Keime ein geeignetes Nährmedium angeboten wird bzw. die Umgebungsbedingungen - beeinflußt z. B. von Sauerstoffgehalt, Temperatur, Licht etc. - das Wachstum fördern. Somit können Keime bis zu einer Grenze von einer koloniebildenden Einheit (KBE) pro Milliliter (ml) nachgewiesen werden. Die relativ unkomplizierte Handhabbarkeit der Platten-Kultur-Methode, die darüber hinaus kein explizites mikrobiologisches Fachwissen erfordert, macht dieses Verfahren auch heute noch bei einer Reihe von Anwendungen zu einer Methode der Wahl.

Nach dem Stand der Technik geht man bei den Kultivierungsverfahren im allgemeinen derart vor, daß Nährmedien (typischerweise Kulturschalen mit Nährmedien auf Basis von Agar-Agar) mit der Probe beimpft werden und bei den jeweiligen Keimen angepaßten, im allgemeinen erhöhten Temperaturen für bis zu einer Woche kultiviert werden (z. B. in einem Brutschrank). Aus dem Wachstum und der Form der entstandenen Kulturen kann ein Fachmann dann die Art und das Ausmaß der mikrobiellen Belastung der Probe ableiten.

Diese Technologie hat den entscheidenden Nachteil, daß sich nur ein unbestimmter Bruchteil der in der Probe enthaltenen Keime kultivieren läßt und die Information erst nach einer Woche zur Verfügung steht.

Um die zuvor beschriebenen Probleme zu lösen, wurden in der Vergangenheit bereits eine Reihe von Verfahren entwickelt, um den mikrobiellen Nachweis zu beschleunigen bzw. die Empfindlichkeit zu erhöhen. Dazu zählen mikroskopische Verfahren, bei denen Keime selektiv oder unselektiv angefärbt und entsprechend nachgewiesen werden, aber auch Verfahren, die auf Immunoassays basieren, und direkte molekularbiologische Verfahren, welche die Erbsubstanz der Keime amplifizieren und anschließend gelelektrophoretisch nachweisen.

Seit einiger Zeit wird versucht, durch neue, sogenannte "Schnellnachweismethoden" die Nachweiszeit von einigen Tagen auf wenige Stunden oder noch darunter zu reduzieren. "Schnellnachweismethoden" werden partiell schon eingesetzt (Impedanz, Biolumineszenz etc.), aber es besteht die Nachfrage nach direkteren und schnelleren Methoden. Denn auch die bisher etablierten "Schnellnachweisverfahren" beruhen auf einer zeitabhängigen Anreicherung von biologischem Material, so daß immer noch 24 bis 48 Stunden für eine Analyse benötigt werden.

Gerätetechnisch aufwendigere Analyseverfahren beruhen beispielsweise auf der Messung der Leitfähigkeit in einer keimhaltigen Lösung. Hierbei verfolgt man die Änderung des Leitwerts, die durch das Keimwachstum und den aus dem Metabolismus generierten Bestandteilen der Lösung hervorgerufen wird. Dieses Impendanz-Verfahren zur Bestimmung des Wechselstromwiderstands hat jedoch den Nachteil, daß sich signifikante Leitwertänderungen erst bei Keimzahlen ab 10³ bis 10⁴ Keime pro ml feststellen lassen. Zwar ist es möglich, auch niedrigere "Start-Keimzahlen" zu analysieren, doch zunächst muß für einen Meßeffekt eine gewisse Keimzahl überschritten werden. Über die Zeit zum Erreichen dieses Schwellwertes kann entsprechend auf die ursprüngliche Konzentration zurückgerechnet werden. Bei sehr niedrigen Keimgehalten muß in der Regel 24 bis 48 Stunden gewartet werden, um ein valides Resultat zu erhalten.

In einem ähnlichen Keimzahlbereich arbeitet das sogenannte ATP-Verfahren (ATP = Adenosintriphosphat), bei dem die im Rahmen einer biochemischen Abbaureaktion durch das ATP der Keime emittierte Biolumineszenz gemessen wird.

Alle drei vorgenannten Verfahren, nämlich Platten-Kultur-, Impendanz- und ATP-Verfahren, können lediglich lebende, vitale Keime nachweisen. Sie alle beruhen darauf, daß sich Keime vermehren und einen funktionierenden Stoffwechsel haben.

Technisch noch anspruchsvollere - und damit auch von der Anschaffung her deutlich teurere - Geräte stellen Durchflußcytometer dar. Bei diesen Apparaten wird die zu analysierende, keimhaltige Lösung durch eine sehr dünne Kapillare gepumpt. Der Durchmesser dieser Kapillare liegt stellenweise im unteren Mikrometerbereich, so daß hier Keime und Zellen vereinzelt betrachtet werden können. Die mit einem Farbstoff markierten Keime werden an diesen Engpässen mit energiereichem, idealerweise monochromatischem Licht (z. B. mit einem Laser) zur Fluoreszenz angeregt. Die Intensität des abgestrahlten Fluoreszenzlichts wird meist mit einem Photomultiplier (PMT) gemessen und einer Pulshöhenanalyse unterzogen (vgl. S. Rapposch et al., "Influence of Fluorescence of Bacteria Stained with Acridine Orange on the Enumeration of Microorganisms in Raw Milk", J. Dairy Sci., 83 (2000), Seiten 2753 bis 2758). Bei geeigneter Auswahl der Farbstoffe ist mit Hilfe der Durchflußcytometer sowohl eine Bestimmung lebender/aktiver als auch toter Keime möglich. Die Verwendbarkeit dieser Durchflußsysteme ist bei höherviskosen und zur Schaumbildung neigenden Proben meist nicht gegeben: Einerseits verstopft die Kapillare leicht, und andererseits ist durch die hervorgerufenen Blasen keine einwandfreie Detektion mit Hilfe des Lasers und des PMT möglich.

Fluoreszenzoptische Verfahren haben in der jüngeren Vergangenheit die herkömmlichen "Schnellnachweismethoden" und Kultivierungsverfahren mehr und mehr abgelöst. Eine Methode, welche die Grundlage der hier beschriebenen Erfindung darstellt, ist die sogenannte Direkte Epifluoreszenz-Filter-Technik, DEFT genannt. Mit der Direkten Epifluoreszenz-Filter-Technik (DEFT) steht eine Direktmethode zur Verfügung, welche auch einen quantitativen "lebend/tot"-Keimnachweis in weniger als einer Stunde erlaubt. Dieses unspezifische, fluoreszenzoptische Verfahren ist als qualitatives Verfahren seit über 25 Jahren in der universitären Grundlagenforschung bekannt (siehe z. B. Pettipher et al., Appl. Environ. Microbiol. 44(4): 809-13, 1982) und hat sich seit Anfang der 1990er Jahre zunehmend in industriellen Anwendungen (z. B. Brauereien, Molkereien, Lebensmittelindustrie etc.) als quantitative Untersuchungsmethode etabliert (Hermida et al., J. AOAC Int. 83(6): 1345-1348, 2000 und Nitzsche et al., Brauwelt, Nr. 5, 177-178, 2000). Es existiert außerdem eine Euro-Vorschrift für die Untersuchung bestrahlter Lebensmittel mit einem DEFT- Screeningverfahren (EN 13783: 2001 "Nachweis der Bestrahlung von Lebensmitteln mit Epifluoreszenz-Filtertechniklaerober mesophiler Keimzahl (DEFT/APC) - Screeningverfahren").

Alternative, selektiv wirkende Fluoreszenzfarbstoffe werden als Laborkits für Vitalnachweise von diversen Herstellern angeboten (z. B. von den Firmen Molecular Probes und EasyProof Laborbedarf GmbH). Einige Anbieter (z. B. die Firma Chemunex) verkaufen Gerätesysteme; die beispielsweise von der Firma Chemunex angebotenen Systeme beruhen entweder auf dem Prinzip der Durchflußcytometrie (L. Philippe, SÖFW-Journal 126, 28-31, 2000), die zur Untersuchung niedriger Keimgehalte einer Anreicherungsphase von 24 Stunden bedarf, oder auf einem mikroskopischen Filtrationsverfahren, welches aber keine "lebend/tot"-Differenzierung erlaubt (Wallner et al., PDA J. Pharm. Sci. Technol. 53(2): 70-74, 1999).

Die Grundidee der DEFT-Methode besteht darin, Keime mit Fluoreszenzfarbstoffen anzufärben, eine Fluoreszenz bei diesen Keimen hervorzurufen und das abgestrahlte Fluoreszenzlicht zu messen. Im Vergleich zum ATP- und Impendanz-Verfahren hat die DEFT-Methode den entscheidenden Vorteil, daß die keimhaltige Probe vor oder nach der Anfärbung filtriert wird. Durch diesen Anreicherungsschritt kann die auch in der Literatur beschriebene Nachweisgrenze auf eine KBE pro ml gesenkt werden. Hinzu kommt, daß mit Hilfe der DEFT-Methode sowohl lebende als auch tote Keime angefärbt werden können. Gerade der Nachweis von Totkeimen spielt bei der Beurteilung des Hygienestatus von Produktionsanlagen (z. B. Biofilmbildung) eine sehr wichtige Rolle. Außerdem kann das Analyseprocedere, d. h. die Filtration und die Anfärbung, innerhalb von ca. 30 Minuten durchgeführt werden. Grundvoraussetzung für die DEFT-Methode ist die Filtrierbarkeit des zu untersuchenden Produkts.

Die Detektion der von den Keimen emittierten Fluoreszenzstrahlung kann auf verschiedene Arten erfolgen: Das am weitesten verbreitete Verfahren ist der Einsatz eines Fluoreszenzmikroskops. Der Membranfilter mit den angefärbten Keimen wird Gesichtsfeld für Gesichtsfeld abgerastert. Dabei wird die Zahl angefärbter Keime vom Mikroskopierenden mit dem bloßen Auge erfaßt und addiert. Diese Vorgehensweise erfordert einerseits ein hohes Maß an mikroskopischer und mikrobiologischer Erfahrung, da eine klare Differenzierung zwischen tatsächlichen Keimen und unvermeidbar auftretenden Fremdpartikeln ähnlicher Größe und Fluoreszenz für den Laien oft sehr schwierig ist. Darüber hinaus stellt dieses Verfahren hohe Anforderungen an die Konzentrationsfähigkeit des Anwenders, weil die Keime auf den nicht ideal planaren Membranfiltern in verschiedenen Tiefenebenen erscheinen und überlappende Gesichtsfelder ohne Doppelzählung klar erkannt werden müssen.

Dem Ziel, dieses Auswerteprocedere zu automatisieren und somit Fehler durch den Bediener weitestgehend auszuschließen, kam man in der Vergangenheit dadurch näher, daß folgende Bestandteile eines Fluoreszenzmikroskops verändert wurden: Der manuell verstellbare Probenhaltertisch wurde durch eine ansteuerbare x,y,z-Einheit ersetzt, die eine Probe automatisiert und genau definiert positionieren kann. Statt des Okulars, das dem Mikroskopierenden zum Betrachten des Objekts dient, werden diverse Kamerasysteme eingesetzt. Diese ermöglichen einerseits eine visuelle Inspektion auf einem Bildschirm, und andererseits liegen digitalisierte Daten vor, die im Nachhinein, z. B. mit Hilfe von Bilderkennungsalgorithmen, analysiert werden können. Weitere Änderungen ergaben sich auch auf Seiten der Lichtquellen zur Fluoreszenzanregung: Neben Weißlichtquellen werden zunehmend leistungsstarke Laser verwendet; diese haben den Vorteil einer sehr definierten Wellenlänge des Anregungslichts, wodurch auf optische Filter nahezu verzichtet werden kann.

Ein simpleres Verfahren, das zwar auf der DEFT-Methode beruht, aber ohne Mikroskop auskommt, ist in der EP 0 713 087 A1 beschrieben. Die EP 0 713 087 A1 beschreibt einen Aufbau und ein Verfahren, bei dem sich angefärbte Keime auf einem festen Trägermaterial befinden. Dieses wird mit einem Laser zeilenförmig abgerastert, wobei die Detektion der emittierten Fluoreszenz bei mindestens einer Wellenlänge erfolgt. Im Vergleich zur klassischen Mikroskopie ermöglicht dieses Verfahren eine deutlich schnellere Analyse, die zudem vollständig automatisiert abläuft. Anhand des beschriebenen Auswertealgorithmus ist diese Methode - obwohl es sich um kein bildgebendes Verfahren im eigentlichen Sinn handelt - in der Lage, Partikel zu zählen, deren Größe zu bestimmen und unter Hinzuziehung der spektralen Charakteristik der einzelnen Teilchen absolute Keimzahlen von weniger als 100 zu detektieren.

Eine Veröffentlichung der Applied Spectrat Imaging, Inc. beschreibt ein Verfahren zur Analyse von FISH-Proben (FISH = Fluorescence in situ hybridisation), das man als eine Art "ortsaufgelöste Spektroskopie" bezeichnen kann (vgl. D. G. Soenksen et al. "Multicolor FISH using a novel spectral bio-imaging system", Proceedings of SPIE (International Society for Optical Engineering), 2678 (1996), Seiten 303 bis 309). Mit Hilfe eines Fluoreszenzmikroskops wird eine farbstoffmarkierte Probe abgerastert und das Bild nach einer Kombination aus Fourier-Transform-(FT)-Spektroskopie mit einer CCD-Kamera pixelweise ausgewertet. So erhält man für jedes Pixel des digitalen Bildes ein komplettes Spektrum vom ultravioletten Bereich bis in den nahen Infrarot-Bereich. Im Gegensatz zu Methoden, bei denen mit unterschiedlichen Filtern gearbeitet wird (siehe z. B. auch I. Ravkin et al." Automated microscopy system for detection and genetic characterization of fetal nucleated red blood cells on slides", Proceedings of SPIE (International Society for Optical Engineering), 3260 (1998), Seiten 180 bis 191) steht so eine viel größere Anzahl von Wellenlängen zur Differenzierung zwischen Keimen und Autofluoreszenz/Fremdpartikein zur Verfügung. Darüber hinaus ermöglicht die FT-Spektroskopie eine zügige Betrachtung der Proben. Nachteilig wirkt sich jedoch die riesige Menge an Daten und deren komplexe Analyse aus.

Das bei I. Ravkin et al., loc. cit., beschriebene Verfahren zur Untersuchung von Zellen ist nur für die Akquisition der Bilder automatisiert; die nachfolgende Auswertung des Bildmaterials erfolgt durch den Bediener. Zur besseren Unterscheidung des von den Teilchen abgestrahlten Fluoreszenzlichts wird ein Filterrad eingesetzt. Dieses Filterrad kann in Abhängigkeit von den zur Anfärbung gewählten Fluoreszenzfarbstoffen konfiguriert werden. Außerdem erläutert diese Referenz ein mögliches Verfahren zur Einstellung eines Autofokus.

Dem für eine erfolgreiche Automatisierung in der Analyse keimhaltiger Proben auf festen Trägern, wie Membranfiltern, wichtigen Aspekt der Autofokuseinstellung widmen sich auch J. Pernthaler et al. "Automated Enumeration of Groups of Marine Picoplankton after Fluorescence In Situ Hybridisation", Appl. Environ. Microbiol., 69 (2003), Seiten 2631 bis 2637. Zum Auffinden des Fokus schlagen die Autoren eine "doppelte Autofokus-Strategie" vor. In einem ersten Schritt wird zunächst der grobe Fokus mit "normal" einfallendem Licht zwischen zwei voneinander unabhängigen Filterstücken eingestellt. Die Autofokussierung verschiedener Gesichtsfelder desselben Filters geschieht allein unter Fluoreszenzbedingungen.

Die WO 2002/064818 A1 bezieht sich auf ein extrem einfaches Vorgehen zum Nachweis von Mikroorganismen: In zwei verschiedenen baulichen Ausführungsformen werden Keime analysiert. Einerseits wird eine mit einem Vital- und Totfarbstoffgemisch versetzte Probe auf einen lichtdurchlässigen Glasträger aufgetropft und die Fluoreszenz nach einer Vergrößerung um den Faktor 220 mit einer CCD-Kamera aufgenommen. In der zweiten Ausführungsform wird die Probe in Analogie zur Durchflußcytometrie durch eine Kapillare gepumpt, mit Licht bestrahlt und das erzeugte Fluoreszenzsignal mit einer Photodiode oder einem Photomultiplier erfaßt. Ungeachtet der wenig differenzierten Betrachtung zu den in den oben zitierten Referenzen genannten Problemen automatischer Analysen, schlagen die Autoren in dieser Anmeldung als Ersatz für eine Xe-Lampe zur Bestrahlung der Probe auch LEDs (light emitting diode) vor. LEDs haben gegenüber "klassischen" Lichtquellen den Vorteil einer hohen Stabilität in der abgestrahlten Lichtleistung. Ferner werden die - immer noch - vorhandenen Nachteile bezüglich der Intensität von LEDs fast jährlich durch Neuentwicklungen kompensiert.

Gerade bei FISH-Proben ergibt sich oftmals die Notwendigkeit zum Einsatz vieler verschiedener Fluoreszenzmarker. Sollen unterschiedliche DNA-Bestandteile eindeutig differenziert werden, ist der Verwendung mehrerer Farbstoffe unerläßlich. Da in diesen Fällen einzelne Filterräder oder Filterwürfel ungeeignet sind, werden zwei oder mehrere Filterwürfel so konfiguriert, daß mindestens ein Filter in beiden gleich ist. Das nun in der WO 98/17992 A2 erläuterte mathematische Verfahren ermöglicht unter Berücksichtigung zweier gleicher Filter in zwei verschiedenen Würfeln eine Angleichung der erhaltenen Datensätze vor allem hinsichtlich der Intensität des abgestrahlten Fluoreszenzlichts. Somit wird die Vergleichbarkeit von Aufnahmen mit unterschiedlichen Filterwürfeln gewährleistet.

A. Pernthaler et al. "Comparison of Fluorescently Labeled Oligonucleotide and Polynucleotide Probes for the Detection of Pelagic Marine Bacteria and Archaea", Appl. Environ. Microbiol., 68 (2992), Seiten 661 bis 667, weisen auf ein wichtiges Phänomen hin, das bei der korrekten Bestimmung der Keimzahl eine Rolle spielt, nämlich das Ausbleichen des Farbstoffs. Bei der in dieser Referenz beschriebenen Untersuchung an Plankton-Proben, die mit verschiedenen Fluoreszenzmarkern versetzt und ausgezählt wurden, hat sich gezeigt, daß vermeintliche Mindergehalte durch ein schnelleres Ausbleichen eines Farbstoffs im Vergleich zu anderen Fluoreszenzsonden erklärt werden können.

Die US 6 122 396 A führt einige der zuvor genannten apparativen und softwaretechnischen Lösungen zu einem Aufbau zusammen. Die Kombination aus Fluoreszenzmikroskop, LED-Beleuchtung und Videokamera wird mit einem spezifischen Bildauswertealgorithmus gekoppelt. In diesem Algorithmus ist ein Referenz-("Trainings-")Datensatz hinterlegt, anhand dessen eine eindeutige Unterscheidung zwischen Mikroorganismen und Fremdpartikeln erfolgt. Parameter, die diesen Referenzdatensatz charakterisieren, sind Morphologie und "Brillanz" der Fluoreszenzstrahlung in einem bestimmten Wellenlängenbereich.

Bei der Untersuchung lebender Zellen unter Verwendung von Farbstoffen rückte in der Vergangenheit die FLIM-Methode (fluorescence lifetime imaging microscopy) in den Mittelpunkt des Interesses. Statt mit ganzen Zellen können ähnliche Experimente auch mit FISH-Proben durchgeführt werden. H. J. Tanke et al. "Use of Platinum Coproporphyrin and Delayed Luminescence Imaging to Extend the Number of Targets FISH Karyotyping", Cytometry 33 (1998), Seiten 453 bis 459, beschreiben eine Methode, bei der durch Messung der Fluoreszenzlebensdauern - also eigentlich eine Lumineszenzmessung - von unterschiedlichen Farbstoffen letztlich auf die Anwesenheit bestimmter DNA-Fragmente zurückgeschlossen werden kann. Je nach Hybridisierung ergeben sich so für DNA-Fragmente charakteristische Intensitäts- und Lebensdauerwerte.

Die sogenannte Membranfilter-Mikrokolonie-Fluoreszenz-Methode (MMCF-Methode, siehe z. B. J. Baumgart, Mikrobiologische Untersuchung von Lebensmitteln, Behr's Verlag 1993, 3. Auflage, Seiten 98 ff.) sieht die Vorbereitung der Probe bzw. der in der Probe vorhandenen Keime auf einem Membranfilter vor. Nachteilig ist dabei, daß eine zeitaufwendige Voranreicherung der Keime vorangehen muß, der Membranfilter für die anschließende Epifluoreszenzmikroskopie vorbehandelt werden muß (Befeuchtung mit speziellen Medien, Dimensionierung und Trocknung) und die Zahl der Keime durch Auszählung der fluoreszenzmarkierten Kolonien unter einem Epifluoreszenzmikroskop oder einer UV-Lampe erfolgen muß.

Einige der zuvor beschriebenen Technologien liefern zwar innerhalb weniger Stunden ein Ergebnis, sie sind aber im allgemeinen sehr aufwendig hinsichtlich der erforderlichen Nachweisgeräte und der nötigen Kenntnisse des Benutzers. Aus diesem Grund haben sich diese Verfahren bisher nicht in ausreichendem Maße im Routineeinsatz etabliert. Weiterhin weisen die einzelnen Methoden Einschränkungen im Hinblick auf Spezifität und Sensitivität auf. Des weiteren besitzen einige der zuvor beschriebenen Methoden eine zu hohe Keimnachweisgrenze, so daß auf eine vorangehende Kultivierung oftmals nicht verzichtet werden kann.

Bei den herkömmlichen Kultivierungsmethoden und bei "Schnellnachweismethoden" mit entsprechenden Anreicherungsschritten sind außerdem durch die Arbeitsweise grundsätzliche Nachteile vorhanden: Die Auswahl der Nährmedien spielt eine entscheidende Rolle, welche Mikroorganismen vermehrt werden können. Selektive Nährmedien bieten hier Vorteile. Aber auch diese können nur die Mikroorganismen vermehren, die physiologisch dazu in der Lage sind. Laut neuesten Erkenntnissen sind jedoch nur 5 % der Mikroorganismen kultivierbar. Daher entstehen mit den konventionellen Methoden oft unzutreffende, negative Ergebnisse, obwohl eine tatsächliche Verkeimung der Probe vorliegt. Darüber hinaus ist die Aussagekraft der Analytik durch die zur Verfügung stehende Zeit begrenzt. Nach Abschluß der Anreicherungszeit müssen alle Keime sich soweit vermehrt haben, daß sie sichtbar geworden sind. Bei verzögertem Anwachsen durch ungünstige Probenahme- oder Anzuchtbedingungen kann es somit zu irrtümlich negativen Ergebnissen kommen. Deshalb erfordert die Durchführung der herkömmlichen Kultivierungsverfahren zum Nachweis von Bakterien und Pilzen oft mehrere Tage, so daß die mikrobiologischen Ergebnisse aber oftmals zu spät kommen, um noch einen regulativen Eingriff in den Produktionsprozeß zu ermöglichen. Mit Nachweisverfahren mittels Kultivierung der Keime lassen sich nur vitale, vermehrungsfähige Keime nachweisen. Oftmals hat aber die bestehende Produktkonservierung Kontaminanten abgetötet. Die im Produkt vorliegenden "toten" Keime lassen sich auf diese Weise jedoch nicht nachweisen, so daß mögliche Hygieneprobleme bei der Produktion oder bei der Abfüllung nicht bemerkt werden bzw. erst dann, wenn Störfälle eintreten, d. h. nach Versagen der Konservierung.

Der Nachweis sehr geringer Keimgehalte, insbesondere unterhalb von 100 KBE pro ml, erfordert entweder eine Wartezeit im Bereich von mehreren Stunden (Impendanz- oder Biolumineszenzverfahren) oder Tagen (Platten-Kultur-Methode) oder aber mikroskopische Verfahren, die langwierig, teuer und ermüdend (vor allen für das menschliche Auge) sind sowie eingewiesenes Fachpersonal erfordern.

Automatisierte Keimnachweise für sehr kleine Keimzahlen wurden technisch schon teilweise realisiert, haben jedoch Nachteile. Bei Durchflußcytometer muß zur Analyse einer größeren Probenmenge (mehrere Milliliter) lange gewartet werden, da die Kapillaren, durch die die Keime fließen, entsprechend dimensioniert sind. Außerdem führt die Verwendung dieser Kapillaren bei höherviskosen Medien zum Verstopfen, und tensidhaltige Produkte erschweren durch Schaum- bzw. Blasenbildung die Analyse.

Beim automatisierten Nachweis von Keimen auf festen Trägern (z. B. Membranfilter) besteht das Hauptproblem einerseits in einer Fokussierung der Objektebene und andererseits in der eindeutigen Unterscheidung zwischen "tatsächlichen" Keimen und unvermeidbar auftretenden Störpartikeln mit Eigenfluoreszenz.

Die auf die Anmelderin selbst zurückgehende DE 102 59 302 A1 bzw. die zu derselben Patentfamilie gehörende WO 2004/055203 A1 beschreibt ein Verfahren zur quantitativen und/oder qualitativen Bestimmung von Keimen in einer Probe, welches einen Verfahrensschritt (a) der Probenaufbereitung mit einer Markierung zumindest eines Teils der in der Probe vorhandenen Keime mittels mindestens eines Fluoreszenzmarkers und einen Verfahrensschritt (b) der quantitativen und/oder qualitativen Detektion und/oder Auswertung umfaßt, wobei die in Verfahrensschritt (b) durchgeführte Detektion und/oder Auswertung fluoreszenzreflexionsphotometrisch erfolgt. Da die fluoreszenzmarkierten Keime nur relativ kurze Zeit einer Bestrahlung ausgesetzt sind, weil die Meßwerte fluoreszenzreflexionsphotometrisch relativ schnell erfaßt werden, wird zwar ein Ausbleichen ("bleaching effect") des Fluoreszenzmarkers und somit eine Verfälschung des Meßergebnisses weitestgehend vermieden; jedoch ermöglicht dieses Verfahren nicht immer eine ausreichend zuverlässige Bestimmung bei extrem geringen Keimkonzentrationen, da Fremd- bzw. Störsignale nicht immer ausreichend von den durch die fluoreszenzmarkierten Keimen generierten Meßsignalen diskriminiert bzw. unterschieden werden können.

Die US 4 444 317 A beschreibt ein Verfahren zur quantitativen und/oder qualitativen Bestimmung von Keimen in einer Probe, das auf dem Ausbleichen der Fluoreszenz zur Diskriminierung zwischen spezifisch fluoreszenmarkierten Keimen und unspezifisch fluoreszenzmarkierten Keimen basiert.

T. Hirschfeld, J. Histochem Cytochem, 1979, 27(1), 96-101 offenbart die Verwendung von Ausbleichverfahren zur Diskriminierung zwischen spezifisch fluoreszenmarkierten Keimen einerseits und Störsignalen auf Grund von intrinsischer Fluoreszenz und freien Fluoreszenzmarkern andererseits.

Der vorliegenden Erfindung liegt somit die Aufgabe zugrunde, ein Verfahren der eingangs genannten Art, welches sich zur quantitativen bzw. qualitativen Bestimmung von Keimen eignet und insbesondere die zuvor geschilderten Nachteile zumindest teilweise vermeidet bereitzustellen.

Die vorliegende Erfindung betrifft somit ein Verfahren zur quantitativen und/oder qualitativen Bestimmung von Keimen in einer Probe mittels Fluoreszenzmarkierung und nachfolgender Detektion und/oder Auswertung wie in Anspruch 1 definiert, wobei die Detektion und/oder Auswertung durch Erfassung und/oder Messung der Fluoreszenzemission erfolgt, wobei die fluoreszenzmarkierten Keime für eine definierte Zeitdauer kontinuierlich einer Anregungsstrahlung einer definierten Wellenlänge oder eines definierten Wellenlängenbereichs ausgesetzt und der zeitabhängige Verlauf der infolge der Anregung erzeugten Fluoreszenzemissionsstrahlung erfaßt wird, so daß eine Diskriminierung zwischen auf die fluoreszenzmarkierten Keime zurückgehenden Meßsignalen einerseits und etwaigen Störsignalen andererseits ermöglicht wird.

Genauer gesagt ist Gegenstand der vorliegenden Erfindung ein Verfahren zur quantitativen und/oder qualitativen Bestimmung von Keimen in einer Probe wie in Anspruch 1 definiert, wobei das Verfahren einen Verfahrenschritt (a) der Probenaufbereitung und einen Verfahrensschritt (b) der Detektion und/oder Auswertung umfaßt, wobei in Verfahrensschritt (a) eine Markierung zumindest eines Teils der in der Probe vorhandenen Keime mittels mindestens eines Fluoreszenzmarkes erfolgt und in Verfahrensschritt (b) eine quantitative und/oder qualitative Detektion und/oder Auswertung erfolgt, wobei die in Verfahrensschritt (b) durchgeführte Detektion und/oder Auswertung durch Erfassung und/oder Messung der Fluoreszenzemission erfolgt, wobei in Verfahrensschritt (b) die in Verfahrensschritt (a) aufbereitete Probe mit den fluoreszenzmarkierten Keimen für eine definierte Zeitdauer kontinuierlich einer Anregungsstrahlung einer definierten Wellenlänge oder eines definierten Wellenlängenbereichs ausgesetzt und der zeitabhängige Verlauf der infolge der Anregung erzeugten Fluoreszenzemissionsstrahlung erfaßt wird, so daß eine Diskriminierung zwischen auf die fluoreszenzmarkierten Keime zurückgehenden Meßsignalen einerseits und etwaigen Störsignalen andererseits ermöglicht wird und auf diese Weise eine quantitative und/oder qualitative Bestimmung der fluoreszenzmarkierten Keime in der Probe erfolgt.

Ein wesentlicher Gedanke der vorliegenden Erfindung ist somit darin zu sehen, daß der zeitabhängige Verlauf der Fluoreszenzemissionsstrahlung bei kontinuierlicher Beleuchtung bzw. Bestrahlung durch die Anregungslichtquelle erfaßt wird und auf diese Weise eine Diskriminierung zwischen auf die fluoreszenzmarkierten Keime zurückgehenden Meßsignalen einerseits und etwaigen Störsignalen andererseits ermöglicht wird. Denn, wie die Anmelderin überraschenderweise herausgefunden hat, zeigen die auf die fluoreszenzmarkierten Keime zurückgehenden Meßsignale einen anderen zeitabhängigen Verlauf der Fluoreszenzemission als die Störsignale, so daß eine Zuordnung der erfaßten bzw. gemessenen Fluoreszenzemissionen auf diese Weise möglich ist, d. h. eine Diskriminierung zwischen Meßsignalen einerseits und etwaigen Störsignalen andererseits ermöglicht wird. Wie die Anmelderin überraschenderweise herausgefunden hat, bleichen fluoreszenzmarkierte Keime im allgemeinen schneller aus als fluoreszierende Fremdpartikel, so daß dieser Effekt für die Zuordnung der Fluoreszenzemissionssignale genutzt werden kann. Auf diese Weise gelingt eine effiziente Differenzierung zwischen auf die fluoreszenzmarkierten Keime zurückgehenden Meßsignalen einerseits und etwaigen Störsignalen andererseits, und zwar auch dann, wenn die Keimanzahl in der Probe äußerst gering ist und folglich die Anzahl der auf die fluoreszenzmarkierten Keime zurückgehenden Meßsignale wesentlich geringer ist als die Anzahl der Störsignale (z. B. um den Faktor 1.000 oder mehr). Auf diese Weise ermöglicht das erfindungsgemäße Verfahren eine relativ einfache, wenig aufwendige Detektion bzw. Auswertung, weil die fluoreszenzmarkierten Keime hierzu im wesentlichen keiner weiteren Aufbereitung bedürften. Insbesondere entfällt ein zeit- und arbeitsaufwendiger Verfahrensschritt der (Vor-)Anreicherung von Keimen, d. h. die erfindungsgemäße Detektion bzw. Auswertung liefert direkt die "authentische" bzw. in der Probe vorhandene Keimzahl.

Unter dem Begriff "zeitabhängiger Verlauf der Fluoreszenzemissionsstrahlung", wie er im Rahmen der vorliegenden Erfindung verwendet wird, wird insbesondere der zeitabhängige Verlauf der Intensität der detektierten bzw. beobachteten Fluoreszenzemission bezeichnet. Hierbei handelt es sich um kein Lumineszenphänomen, da dort erst nach dem Abschalten der Anregungslichtquelle die Intensität der Fluoreszenz verfolgt wird.

Der Begriff "Störsignale" bezeichnet alle detektierten bzw. gemessenen Fluoreszenzemissionssignale, welche nicht auf die fluoreszenzmarkierten Keime zurückgehen. Solche Störsignale können beispielsweise durch Fremdstoffe mit intrinsischer Fluoreszenz, insbesondere Kunststoffpartikel, in der Probe erzeugt werden, aber auch durch unspezifische Bindungen des Fluoreszenzmarkers an Fremdpartikel oder aber auch durch Verunreinigungen aufgrund von freien, z. B. nicht ausgewaschenen Fluoreszenzmarkern.

Das erfindungsgemäße Verfahren ermöglicht grundsätzlich sowohl die qualitative wie auch die quantitative Bestimmung von Keimen in einer Probe, d. h. sowohl die Bestimmung der Art bzw. Spezies von Keimen überhaupt als auch deren Anzahl bzw. deren Konzentration.

Das erfindungsgemäße Verfahren wird derart durchgeführt, daß der zeitabhängige Verlauf der infolge der Anregung erzeugten Fluoreszenzemissionsstrahlung dahingehend erfaßt wird, daß die Kinetik des Abbaus bzw. Ausbleichens des Fluoreszenzmarkers bzw. der fluoreszenzmarkierten Keime (d. h. Fluoreszenzmarker mit Bindung an die Keime beim Nachweis von toten Keimen bzw. Fluoreszenzmarker, die durch den Metabolismus lebender Keime umgewandelt werden) erfaßt wird. Wie zuvor geschildert, nutzt man im Rahmen des erfindungsgemäßen Verfahrens die überraschende Erkenntnis bzw. Tatsache, daß die fluoreszenzmarkierten Keime ein anderes Abbau- bzw. Ausbleichverhalten des Fluoreszenzmarkers aufweisen als Fremdpartikel, insbesondere im allgemeinen schneller ausbleichen als Fremdpartikel. Über die Erfassung der Kinetik des Abbaus und/oder des Ausbleichens des Fluoreszenzmarkers läßt sich - in Abgleichung mit den Störsignalen - eine Zuordnung der detektierten bzw. erfaßten Fluoreszenzemissionssignale zu den zu bestimmenden Keimen einerseits und den Störsignalen andererseits realisieren, so daß eine gezielte Diskriminierung bzw. Differenzierung ermöglicht wird, die eine zuverlässige quantitative und/oder qualitative Bestimmung der fluoreszenzmarkierten Keime - auch bei extrem geringen Konzentrationen - ermöglicht.

Die Erfassung der Kinetik des Abbaus und/oder Ausbleichens der Fluoreszenzemissionssignale bzw. des Fluoreszenzmarkers und der hiermit markierten Keime erfolgt im allgemeinen über die Erfassung des zeitabhängigen Verlaufs der Intensitäten der Fluoreszenzemissionssignale.

Auf Basis der durch Fluoreszenzemission ermittelten Meßwerte kann dann die in der Probe vorhandene Zahl an Keimen bestimmt werden, gegebenenfalls mittels geeigneter Kalibrierung.

Die Formulierung "Markierung zumindest eines Teils der in der Probe vorhandenen Keime" meint insbesondere folgendes: Je nachdem, ob nur spezielle in der Probe vorhandene Keime bzw. Keimarten oder aber sämtliche in der Probe vorhandenen Keime bestimmt werden sollen, markiert man im ersteren Fall gezielt nur ein speziellen Teil der in der Probe vorhandenen Keime (im allgemeinen mit keimspezifischen Fluoreszenzmarkern), während man im letzteren Fall sämtliche in der Probe vorhandenen Keime markiert (im allgemeinen mit keimunspezifischen Fluoreszenzmarkern oder aber einer Mischung verschieden keimspezifischer Fluoreszenzmarker).

Nach dem erfindungsgemäßen Verfahren lassen sich dann aufgrund der ermittelten Meßwerte, gegebenenfalls mittels geeigneter Kalibrierung, die in der Probe vorhandene Zahl an Keimen bestimmen (und zwar im Fall der Fluoreszenzmarkierung sämtlicher in der Probe vorhandenen Keime die Gesamtzahl aller in der Probe vorhandenen Keime und im Fall der Fluoreszenzmarkierung nur spezieller Keime deren Gesamtzahl). Wie im folgenden noch erläutert, ermöglicht der Einsatz keimspezifischer Fluoreszenzmarker darüber hinaus auch eine qualitative Aussage in bezug auf das Vorhandensein spezieller Keime in der Probe.

Die Reproduzierbarkeit bzw. Zuverlässigkeit des erfindungsgemäßen Verfahrens läßt sich noch weiter dadurch erhöhen, daß in mindestens zwei zeitlich aufeinanderfolgenden Intervallen die Fluoreszenzstrahlung bei jeweils voneinander unterschiedlichen, aber jeweils definierten Wellenlängen oder Wellenlängenbereichen von der aufbereiteten Probe mit den fluoreszenzmarkierten Keimen detektiert wird.

Eine weitere Steigerung der Leistungsfähigkeit des erfindungsgemäßen Verfahrens läßt sich dadurch erreichen, daß zusätzlich eine Diskriminierung zwischen auf die fluoreszenzmarkierten Keime zurückgehenden Meßsignale einerseits und etwaigen Störsignalen andererseits über die Analyse der Fluoreszenzcharakteristik erfolgt. Dabei wird insbesondere das Verhältnis der Fluoreszenzintensitäten bei verschiedenen, aber definierten Wellenlängen oder Wellenlängenbereichen erfaßt, so daß eine zuverlässige Diskriminierung zwischen auf die zu bestimmenden Keime zurückgehenden Meßsignalen einerseits und etwaigen Störsignalen andererseits ermöglicht wird.

Die Wellenlänge bzw. der Wellenlängenbereich der Anwendungsstrahlung sollte auf die Fluoreszenzcharakteristik des Fluoreszenzmarkers bzw. der fluoreszenzmarkierten Keime abgestimmt sein, insbesondere auf deren Absorptionsmaxima in bezug auf die Fluoreszenzemission.

Die Leistungsfähigkeit des erfindungsgemäßen Verfahrens wird auch dadurch noch weiter erhöht, daß - zusätzlich zu der Diskriminierung über die Erfassung des zeitabhängigen Verlaufs der Fluoreszenzemissionsstrahlung - eine Diskriminierung über die Größe und/oder Form der emittierenden Partikel erfolgt, was beispielsweise im Rahmen automatisierter Bilderfassungsverfahren erfolgen kann. Über die Erfassung der Größe und/oder Form der emittierenden Partikel ergibt sich - zusammen mit der Beurteilung des zeitabhängigen Verlaufs - ein weiteres Kriterium für eine zuverlässige Zuordnung zu Stör- bzw. Fremdpartikeln einerseits und zu den zu bestimmenden Keimen andererseits. Da die nachzuweisenden Keime im allgemeinen kleiner als 6 µm sind, können alle größeren Signale vernachlässigt werden. In diesem Zusammenhang ist auch die Form zu sehen; denn im allgemeinen ist das Verhältnis der Hauptachsen bei Keimen unter der erfindungsgemäßen Verwendung eines 10fach- oder 20fach-Mikroskopobjektivs näherungsweise 1 : 1, d. h. die Keime erscheinen - mit Ausnahme der Hyphenform eines Pilzes - als runde Gebilde. Partikel, die dagegen ein anderes Hauptachsenverhältnis aufweisen, werden somit außer acht gelassen.

Gemäß einer besonders bevorzugten Ausführungsform des erfindungsgemäßen Verfahrens erfolgt die Diskriminierung zwischen auf die fluoreszenzmarkierten Keime zurückgehenden Meßsignalen einerseits und etwaigen Störsignalen andererseits dreistufig, d. h. unter Anwendung aller drei zuvor genannten Diskriminierungsmethoden, nämlich sowohl über die Erfassung des zeitabhängigen Verlaufs der Fluoreszenzemissionsstrahlung als auch über die Größe und/oder Form der emittierenden Partikel sowie schließlich über die Analyse der Fluoreszenzcharakteristik.

Für die Durchführung des erfindungsgemäßen Verfahrens bzw. die Detektion und/oder Auswertung werden die zu bestimmenden Keime im allgemeinen auf einem Träger aufgebracht bzw. fixiert. Zumindest für die Dauer der Messung liegen die zu bestimmenden Keime auf diese Weise in sozusagen immobilisierter Form insofern vor, als sie auf dem Träger befindlich fixiert sind und ihre Position nicht ändern können, so daß eine zuverlässige Signalzuordnung möglich ist.

Die Probenaufbereitung erfolgt im allgemeinen, wie dies in den auf die Anmelderin selbst zurückgehenden Druckschriften DE 102 59 302 A1 und WO 2004/055203 A1 beschrieben ist.

Im allgemeinen werden die fluoreszenzmarkierten Keime bei der im Verfahrensschritt (a) durchgeführten Probenaufbereitung auf einem vorzugsweise porösen Träger (z. B. einen Membranfilter, wie beispielsweise einem Polycarbonatmembranfilter, oder einem Siliciummikrosieb) aufgebracht, wobei der Träger vorzugsweise porös ausgebildet ist. Der Träger, insbesondere Membranfilter oder Siliciummikrosieb, sollte im allgemeinen derart ausgebildet sein, daß er die Keime zurückhält bzw, in bezug auf die Keime undurchlässig ist. Zu diesem Zweck sollte die Größe der Poren des Trägers so gewählt sein, daß die Porengröße kleiner als die Größe der in der Probe vorhandenen Keime ist. Als poröse Trägermaterialien können erfindungsgemäß beispielsweise Membranfilter verwendet werden, beispielsweise Membranfilter auf Basis von Polycarbonat, PTFE, Polyestern, Cellulose und Cellulosederivaten, wie Celluloseacetat, regenerierter Cellulose, Nitrocellulose oder Cellulosemischester. Erfindungsgemäß geeignete Membranfilter werden beispielsweise von der Firma Macherey-Nagel vertrieben (z. B die "PORAFIL^{®}"-Serie). Als poröser Träger kann auch ein Siliciummikrosieb verwendet werden, welches eine besonders glatte und ebenmäßige Oberfläche aufweist, weshalb sich darauf befindliche Keime noch besser detektieren lassen; ferner ist die Reinigung solcher Siebe relativ einfach zu bewerkstelligen, und solche Siebe sind mehrfach verwendbar, darüber hinaus besitzen die Siliciummikrosiebe eine gute Biokompatibilität und eine starre Struktur, die erhebliche Vorteile bei ihrer Handhabung bewirkt.

Der Einsatz eines porösen Trägers, insbesondere Membranfilters oder Siliciummikrosiebs, bietet den Vorteil, daß die Detektion bzw. Auswertung insbesondere ohne weitere Probenbehandlung, -aufbereitung, -übertragung oder dergleichen (d. h. insbesondere ohne Voranreichung) unmittelbar auf dem porösen Träger erfolgen kann.

Vorteilhafterweise wird der in dem erfindungsgemäßen Verfahren eingesetzte Fluoreszenzmarker derart ausgewählt wird, daß er membrangängig in bezug auf den bei der in Verfahrensschritt (a) durchgeführten Probenaufbereitung verwendeten Träger, insbesondere Membranfilter oder Siliciummikrosieb, ist. Dies hat den Vorteil, daß bei der Detektion bzw. Auswertung keine durch überschüssige Fluoreszenzmarker verursachten Störsignale bzw. kein Hintergrundrauschen auftritt und folglich ein günstiges Signal/Hintergrund-Verhältnis bzw. Signal/Rausch-Verhältnis erzielt wird.

Die Durchführung der Fluoreszenzmarkierung der in der Probe vorhandenen Keime in Verfahrensschritt (a) des erfindungsgemäßen Verfahrens wird in an sich bekannter Weise durchgeführt. Dies ist dem Fachmann ohne weiteres geläufig. Beispielsweise können zu diesem Zweck die zu markierenden Keime mit einer Lösung oder Dispersion der im Überschuß in bezug auf die vorhandenen Keime vorliegenden Fluoreszenzmarker in Kontakt gebracht werden, wobei die Kontaktzeit ausreichend sein muß, um eine vollständige Fluoreszenzmarkierung aller Keime zu gewährleisten, die bei diesem Verfahrensschritt markiert werden sollen (je nach Auswahl des Fluoreszenzmarkers z. B. sämtliche in der Probe vorhandenen Keime oder aber sämtliche Keime nur einer oder mehrerer Keimarten). Nach der eigentlichen Fluoreszenzmarkierung können dann die überschüssigen Fluoreszenzmarker entfernt bzw. von den fluoreszenzmarkierten Keimen abgetrennt werden. Dies kann im Fall der Verwendung eines porösen Trägers, insbesondere Membranfilters oder Siliciummikrosiebs, der membrangängig in bezug auf die Fluoreszenzmarker, aber undurchlässig in bezug auf die Keime ist, beispielsweise dadurch geschehen, daß man (z. B. durch Anlegen von Überdruck oder Unterdruck) die Lösung bzw. Dispersion der überschüssigen Fluoreszenzmarker über den porösen Träger abführt und gegebenenfalls das Ganze mit Wasser, Pufferlösungen oder anderen Flüssigkeiten nachspült, so daß auf dem Träger schließlich nur noch die fluoreszenzmarkierten Keime (gegebenenfalls zusammen mit den Keimen, die gezielt unmarkiert geblieben sind) verbleiben. Dies ermöglicht dann eine sich ohne weiteres in Verfahrensschritt (b) des erfindungsgemäßen Verfahrens anschließende Detektion bzw. Auswertung.

Gegebenenfalls kann der Verfahrensschritt (a) der Probenaufbereitung auch eine Inaktivierung und/oder Entfernung von gegebenenfalls in der Probe vorhandenen keimhemmenden und/oder keimtötenden Substanzen oder Bestandteilen (z. B. Konservierungsstoffe, Tenside etc.) umfassen. Dies verhindert, daß die späteren Meßergebnisse dadurch verfälscht werden, daß ein Teil der Keime während der Probenaufbereitung oder Messung durch die keimhemmenden bzw. keimtötenden Substanzen oder Bestandteilen abgetötet bzw. inaktiviert werden und somit eine fälschlich zu geringe Keimzahl bestimmt wird. Auf diese Weise wird eine Bestimmung der Keimzahl auch in Proben mit keimhemmenden und/oder keimtötenden Substanzen oder Bestandteilen (z. B. Konservierungsstoffe, Tenside etc.) ermöglicht, so daß das erfindungsgemäße Verfahren beispielsweise auch bei Tensid- und Dispersionsprodukten angewendet werden kann.

Der je nach Art der Probe nur gegebenenfalls durchgeführte Verfahrensschritt der Inaktivierung bzw. Entfernung von gegebenenfalls in der Probe vorhandenen keimhemmenden bzw. keimtötenden Substanzen oder Bestandteilen wird vorteilhafterweise vor der Fluoreszenzmarkierung, vorzugsweise unmittelbar nach der Probenahme bzw. unmittelbar zu Beginn der in Verfahrensschritt (a) des erfindungsgemäßen Verfahrens durchgeführten Probenaufbereitung ausgeführt; auf diese Weise ist sichergestellt, daß die keimhemmenden bzw. keimtötenden Substanzen, Bestandteile, Inhaltsstoffe und dergleichen im wesentlichen noch keine Änderung der in der ursprünglichen Probe vorhandenen Keimzahl bewirkt haben können. Gleichermaßen, wenn auch weniger bevorzugt, ist es möglich, die Inaktivierung bzw. Entfernung von gegebenenfalls in der Probe vorhandenen keimhemmenden bzw. keimtötenden Substanzen oder Bestandteilen nach der Fluoreszenzmarkierung durchzuführen. Ebenfalls ist es möglich, die Fluoreszenzmarkierung und die Inaktivierung bzw. Entfernung der keimhemmenden bzw. keimtötenden Substanzen oder Bestandteilen gleichzeitig durchzuführen.

Der je nach Art der Probe nur gegebenenfalls durchgeführte Verfahrensschritt der Inaktivierung bzw. Entfernung von gegebenenfalls in der Probe vorhandenen keimhemmenden bzw. keimtötenden Substanzen oder Bestandteilen erfolgt in an sich bekannter Weise. Beispielsweise kann auf den Beitrag von Stumpe et al. "Chemolumineszenz-basierte Direktnachweise von Mikroorganismen - Ein Erfahrungsbericht aus der Lebensmittel- und Kosmetikindustrie" auf den Seiten 317 bis 323 des Tagungsbandes "HY-PRO 2001, Hygienische Produktionstechnologie/Hygienic Production Technology", 2. Internationaler Fachkongreß und Ausstellung, Wiesbaden 15.-17.5.2001 und die in diesem Beitrag angegebene Literatur verwiesen werden. Im allgemeinen kann dies dadurch erfolgen, daß die zu analysierende Probe mit einer geeigneten Inaktivierungs- und/oder Konditionierlösung in Kontakt gebracht wird. Solche Inaktivierungs- bzw. Konditionierlösungen sind dem Fachmann an sich bekannt (z. B. wäßrige THL-Konditionierlösung, TLH = Tween-Lecithin-Histidin). Erfindungsgemäß geeignete wäßrige Inaktivierungs- bzw. Konditionierlösungen können beispielsweise neben TLH (z. B. Polysorbat 80 = Tween 80, Soyalecithin und L-Histidin) auch noch Puffersubstanzen (z. B. Phosphatpuffer, wie Hydrogenphosphat und/oder Dihydrogenphosphat), weitere Salze (z. B. Natriumchlorid und/oder Natriumthiosulfat) und Trypton (Pepton aus Casein) enthalten.

Eine erfindungsgemäß besonders geeignete Inaktivierungs- bzw. Konditionierlösung hat die folgende Zusammensetzung:
- Trypton 1,0 g
- Natriumchlorid 8,5 g
- Natriumthiosulfatpentahydrat 5,0 g
- 0,05 M Phosphatpufferlösung 10 ml
- TLH-Wasser ad 1000 ml.

Das erfindungsgemäß einsetzbare TLH-Wasser hat insbesondere folgende Zusammensetzung:
- Polysorbat 80 (Tween 80) 30,0 g
- Soyalecithin 3,0 g
- L-Histidin 1,0 g
- Vollentsalztes Wasser ad 1000 ml.

Die erfindungsgemäß einsetzbare Phosphatpufferlösung hat insbesondere folgende Zusammensetzung:
- Kaliumdihydrogenphosphat 6,8045 g
- Dikaliumhydrogenphosphat 8,709 g
- Vollentsalztes Wasser ad 1000 ml.

Die Auswahl an Fluoreszenzmarker(n) ist nicht kritisch. Hier können, je nach Anwendung und Art der Keime, die aus dem Stand der Technik an sich bekannten Fluoreszenzmarker verwendet werden, sofern sie sich zur Verwendung im Rahmen des erfindungsgemäßen Verfahrens eignen.

Der Begriff "Fluoreszenzmarker" wird im Rahmen der vorliegenden Erfindung sehr breit verstanden und meint insbesondere jeden Fluoreszenzmarker, der derart ausgebildet ist, daß er mit den Keimen in Wechselwirkung tritt, beispielsweise an die Keime, insbesondere an deren Zellwand (Hülle) und/oder Nukleinsäure, anbindet und/oder von den Keimen aufgenommen, insbesondere metabolisiert und/oder enzymatisch umgesetzt wird.

Bei dem erfindungsgemäß eingesetzten Fluoreszenzmarker kann es sich beispielsweise um einen keimunspezifischen Fluoreszenzmarker oder ein Gemisch keimunspezifischer Fluoreszenzmarker handeln. Dies ermöglicht eine relativ kostengünstige Fluoreszenzmarkierung aller in der Probe vorhandenen Keime und somit eine relativ schnelle Bestimmung der Gesamtkeimzahl in der Probe.

Insbesondere für den Fall, daß selektiv nur spezielle Keime qualitativ und quantitativ erfaßt werden sollen, kann als Fluoreszenzmarker ein keimspezifischer Fluoreszenzmarker oder ein Gemisch verschieden keimspezifischer Fluoreszenzmarker eingesetzt werden.

Gleichermaßen kann als Fluoreszenzmarker ein Gemisch keimunspezifischer und keimspezifischer Fluoreszenzmarker eingesetzt werden.

Beispielsweise kann als Fluoreszenzmarker auch ein mit lebenden Keimen in Wechselwirkung tretender Fluoreszenzmarker eingesetzt werden. Gleichermaßen kann als Fluoreszenzmarker auch ein mit "toten" Keimen in Wechselwirkung tretender Fluoreszenzmarker eingesetzt werden.

Ebenfalls ist es möglich, als Fluoreszenzmarker ein Gemisch von mit lebenden Keimen in Wechselwirkung tretenden Fluoreszenzmarkern und mit "toten" Keimen in Wechselwirkung tretenden Fluoreszenzmarkern einzusetzen. Auf diese Weise kann eine lebend/tot-Differenzierung der in der Probe vorhandenen Keime erreicht werden. Solche Gemische sind aus dem Stand der Technik an sich bekannt (siehe beispielsweise Stumpe et al., loc. cit., und das dort genannten System der Firma EasyProof Laborbedarf GmbH, Voerde).

Das zuvor genannte Markersystem der Firma EasyProof Laborbedarf GmbH wurde ursprünglich für die Brauereiindustrie eingeführt (Eggers et al., Brauindustrie 6, 34-35, 2001). Dabei wird eine nichtfluoreszierende Vorstufe (d. h. ein Vorläufer oder Precursor) eines Fluoreszenzmarkers in eine intakte mikrobielle Zelle aufgenommen und durch enzymatische Aktivität (Esterase) innerhalb der Zelle (im Cytoplasma) in eine fluoreszierende Verbindung umgewandelt (Grünfärbung = Lebendnachweis); damit dieser Vorgang ablaufen kann, muß eine intakte Zellmembran mit Membranpotential vorhanden sein. Der Nachweis "toter" Zellen erfolgt über die Einlagerung eines spezifischen Fluoreszenzfarbstoffes in die DNA der Zelle. Dieser Einbau kann wiederum nur bei Zellen erfolgen, die eine defekte Zellmembran aufweisen (Rotfärbung = Totnachweis). Da beide Reaktionen auf unterschiedlichen Prinzipien beruhen, sind die Ergebnisse voneinander unabhängig. Diese Markierungstechnik schädigt die Zellen nicht, so daß bei der Auswertung der aktuelle mikrobiologische Status der Probe bestimmt werden kann.

Bei dem erfindungsgemäßen Verfahren lassen sich als Fluoreszenzmarker beispielsweise auch die in der Epifluoreszenzmikroskopie oder in der DEFT (Direkte Epifluoreszenz-Filter-Technik) oder in der MMCF (Membranfilter-Mikrokolonie-Fluoreszenz-Methode) üblicherweise zur Keimmarkierung verwendeten Fluoreszenzmarker einsetzen.

Beispielsweise kann als Fluoreszenzmarker ein Fluoreszenzfarbstoff oder aber eine Vorstufe eines solchen Fluoreszenzfarbstoffs, aus dem durch Wechselwirkung mit den Keimen, insbesondere durch Metabolisierung und/oder enzymatische Umsetzung, der Fluoreszenzfarbstoff generiert wird, eingesetzt werden.

Beispiele für derartige Vorstufen von Fluoreszenzfarbstoffen sind z. B. in der WO 86/05206 A1 und in der EP 0 443 700 A2 beschrieben (z. B. nichtfluoreszierendes Diacetylfluorescein, das enzymatisch zu Fluorescein umgesetzt werden kann)

Beispiele für erfindungsgemäß als Fluoreszenzmarker verwendbare Fluoreszenzfarbstoffe sind, ohne Beschränkung, z. B. 3,6-Bis[dimethylamino]acridin (Acridinorange), 4',6-Diamido-2-phenylindol (DAPI), 3,8-Diamino-5-ethyl-6-phenyl-phenanthridiniumbromid (Ethidiumbromid), 3,8-Diamino-5-[3-(diethyl-methylammonio)propyl]-6-phenyl-phenanthridiniumdüodid (Propidiumiodid), Rhodamine wie Rhodamin B und Sulforhodamin B sowie Fluoresceinisothiocyanat. Für weitere Beispiele kann auch auf die EP 0 940 472 A1 oder aber auf Molecular Probes' Handbook of Fluorescent Probes and Research Chemicals, 5. Ausgabe, Molecular Probes Inc., Eugene, Oregon (P. R. Haugland, Editor, 1992) verwiesen werden. Des weiteren kann auch auf die einschlägigen Chemikalienkataloge verwiesen werden (z. B. Katalog Biochemikalien und Reagenzien für die Life Science Forschung der Firma Sigma-Aldrich, "Fluorescent Labeling Reagents", Ausgabe 2002/2003).

Bei dem erfindungsgemäßen Verfahren lassen sich als Fluoreszenzmarker beispielsweise auch Nukleinsäuresonden (z. B. keimspezifische Nukleinsäuresonden) einsetzen, die ihrerseits fluoreszenzmarkiert sind, insbesondere mit einer fluoreszierenden Gruppe oder einem fluoreszierenden Molekül. Die fluoreszierende Gruppe oder das fluoreszierende Molekül können beispielsweise kovalent oder anderweitig an die Nukleinsäuresonde gebunden sein. Bei der erfindungsgemäß als Fluoreszenzmarker eingesetzten Nukleinsäuresonde kann es sich beispielsweise um ein fluoreszenzmarkiertes Oligo- oder Polynukleotid oder eine fluoreszenzmarkierte DNA- oder RNA-Sonde handeln. Im allgemeinen werden aus Stabilitätsgründen DNA-Sonden erfindungsgemäß bevorzugt.

Beispiele für erfindungsgemäß als Fluoreszenzmarker einsetzbare Nukleinsäuresonden sind beispielsweise die in der WO 01/85340 A2, WO 01/07649 A2 und WO 97/14816 A1 genannten Sonden.

So können beispielsweise als Nukleinsäuresonden die in der Fluoreszenz-in-situ-Hybridisierung (FISH) üblicherweise zur Markierung (DNA- oder RNA-Markierung) verwendeten Nukleinsäuresonden eingesetzt werden. Für weitere diesbezügliche Einzelheiten kann auf RÖMPP Lexikon Biotechnologie und Gentechnik, 2. Auflage, Georg Thieme Verlag Stuttgart, Seiten 285/286, Stichwort: "FISH" und die dort angegebene Literatur sowie auf die WO 01/07649 A2 verwiesen werden.

Gleichermaßen kann als Fluoreszenzmarker auch ein insbesondere keimspezifischer Antikörper eingesetzt werden, der seinerseits fluoreszenzmarkiert ist, insbesondere mit einer fluoreszierenden Gruppe oder einem fluoreszierenden Molekül, wobei die fluoreszierende Gruppe oder das fluoreszierende Molekül kovalent oder anderweitig an den Antikörper gebunden sein kann.

Die Menge bzw. Konzentration an eingesetzten Fluoreszenzmarkern wird der Fachmann den jeweiligen Gegebenheiten des Einzelfalls anpassen. Dies ist ihm ohne weiteres geläufig. Beispielsweise sollte bei einer lebend/tot-Differenzierung der in der Probe vorhandenen Keime für eine gute Keimanfärbung bei gleichzeitig schwacher "Hintergrundfärbung" ein geeignetes Mischungsverhältnis von "Lebendfarbstoff/"Totfarbstoff" gewählt werden; dies im Einzelfall auszuwählen, liegt im Rahmen des fachmännischen Könnens.

Im allgemeinen liegt die Nachweisegrenze bei dem erfindungsgemäßen Verfahren in bezug auf die zu bestimmenden Keime bei ≤ 100 Koloniebildende Einheiten (KBE) pro Milliliter Probevolumen, vorzugsweise bei ≤ 10 Koloniebildende Einheiten (KBE) pro Milliliter Probevolumen. Daher kommt das erfindungsgemäße Verfahren ohne jegliche Voranreicherung aus. Die niedrige Nachweisgrenze ist beispielsweise zur Erfüllung bestimmter Richtlinien oder Vorschriften von entscheidender Bedeutung. So muß beispielsweise laut CTFA-Richtlinie für kosmetische Rohstoffe bei deutlich höher liegender Keimzahlgrenze (z. B. 10² bis 10³ KBE/ml) eine zeitaufwendige und kostenintensive Abwesenheitsprüfung von bestimmten Problemkeimen, d. h. pathogenen Keimen, durchgeführt werden.

Im allgemeinen lassen sich mit dem erfindungsgemäßen Verfahren Keimzahlen im Bereich von etwa 10 KBE pro Milliliter Probevolumen oder sogar weniger bis etwa 10⁸ KBE pro Milliliter Probevolumen bestimmen. Dabei sollte zu Zwecken quantitativer Auswertungen ab einer bestimmten Keimzahl (im allgemeinen ab ca. 10² KBE pro Milliliter Probevolumen) die Probe vorab entsprechend, d. h. in geeigneter Weise, verdünnt werden.

Das erfindungsgemäße Verfahren eignet sich prinzipiell für die Bestimmung beliebiger Keime, insbesondere pathogener Keime aller Art (z. B. Mikroorganismen aller Art, insbesondere einzellige Mikroorganismen, wie Bakterien und Pilze, z. B. Hefen oder Schimmelpilze).

Das erfindungsgemäße Verfahren eignet sich prinzipiell zur quantitativen und/oder qualitativen Bestimmung von Keimen in beliebigen Produkten (d. h. Medien, Matrices, Lösungen etc.), vorzugsweise filtrierbaren, insbesondere flüssigen und/oder fließfähigen Produkten. Bei festen oder als solchen nicht filtrierbaren Produkten müssen diese bei der Probenaufbereitung in eine dem erfindungsgemäßen Verfahren zugängliche Form überführt werden; dies geschieht mit an sich bekannten Methoden, beispielsweise durch Überführen in eine Lösung oder Dispersion, Zerkleinerung, Extraktion etc.

Beispielsweise eignet sich das erfindungsgemäße Verfahren zur quantitativen und/oder qualitativen Bestimmung von Keimen in Lebensmitteln, tensidhaltigen Produkten wie Wasch- und Reinigungsmitteln, Mitteln zur Oberflächenbehandlung, Dispersionsprodukten, Kosmetika, Hygieneprodukten und Produkten der Körperpflege, Pharmazeutika, Klebstoffen, Kühlschmierstoffen, Lacken und (Lack-)Koagulationen sowie Rohstoffen und Ausgangsstoffen für die vorgenannten Produkte.

Das erfindungsgemäße Verfahren eignet sich damit für alle Arten möglicher Rohstoffe, Zwischen- und Endprodukte aus den unterschiedlichen Bereichen, wie z.B. Lebensmittel, Markenartikel, Kosmetika, Klebstoffe, Kühlschmierstoffe (z. B. ölige Kühlschmierstoffemulsionen); Prozeßflüssigkeiten von Anlagen etc., mit der Einschränkung, daß die nachzuweisenden Keime sich über ein Trennverfahren, wie Filtration oder Sedimentation, abtrennen lassen sollten. Dabei ist es auch nicht von Bedeutung, ob die Produkte in fester oder flüssiger Form vorliegen.

Aufgrund der relativ einfachen Durchführbarkeit und der besonderen Kombination von Verfahrensschritten eignet sich das erfindungsgemäße Verfahren insbesondere für die automatisierte Durchführung (z. B. im Rahmen von Produktions- und/oder Qualitätskontrolle). Neben der Produktions- und/oder Qualitätskontrolle (z. B. bei der Abfüllung von Tensid-Flüssigprodukten, Dispersionen, konservierten Produkten etc.) eignet sich das erfindungsgemäße Verfahren beispielsweise auch zur Untersuchung von Störfällen bzw. Kontaminationen zur Bestimmung des Keimstatus oder auch zur Bewertung von Maßnahmen zur Produktsanierung oder aber auch zur Optimierung bzw. Überprüfung von Anlagenreinigungen (z. B. in Anlagen zur Herstellung konservierter Produkte), beispielsweise im Rahmen von CIP-Verfahren (CIP = Cleaning in Place) und SIP-Verfahren (SIP = Sterilisation in Place).

Das erfindungsgemäße Verfahren wird üblicherweise wie folgt durchgeführt:

Die Probeaufbereitung kann, wie in der DE 102 59 302 A1 und der WO 2004/055203 beschrieben, durchgeführt werden. Dies kann beispielsweise wie folgt geschehen:

Die Probe mit den quantitativ und/oder qualitativ zu bestimmenden Keimen wird in ein geeignetes Probengefäß eingebracht, dessen Boden mit einem im allgemeinen porösen Träger, z. B. einem Membranfilter oder einem Siliciummikrosieb, versehen ist und das keimfrei verschließbar sein sollte. Der poröse Träger liegt mit seinem äußeren Rand auf dem Probengefäß auf, so daß der äußere, konzentrische Rand nicht mit Keimen belegt wird. Falls eine Probe untersucht werden soll, die keimhemmende bzw. keimtötende Substanzen oder Bestandteile (z. B. Konservierungsstoffe oder Tenside) aufweist, wird zunächst eine Inaktivierung und/oder Entfernung dieser Substanzen bzw. Bestandteile durchgeführt, indem die Probe mit einer geeigneten Inaktivierungs- und/oder Konditionierlösung in Kontakt gebracht wird, und zwar für eine Zeitdauer die ausreicht, um die Inaktivierung und/oder Entfernung dieser Substanzen bzw. Bestandteile zu ermöglichen. Mittels Über- oder Unterdruck wird die Inaktivierungs- und/oder Konditionierlösung über den Membranfilter entfernt. Anschließend wird gegebenenfalls durch einfaches oder mehrfaches Waschen mit Wasser überschüssige bzw. verbleibende Inaktivierungs- und/oder Konditionierlösung über den Membranfilter entfernt, und zwar üblicherweise durch Anlegen eines Über- oder Unterdrucks, so daß auch das Waschwasser auf einfache Art entfernt wird. Hieran schließt sich eine Markierung zumindest eines Teils der in der Probe vorhandenen Keime mittels mindestens eines Fluoreszenzmarkers an. Zu diesem Zweck können die Keime beispielsweise mit einer Lösung oder Dispersion des Fluoreszenzmarkers in Kontakt gebracht werden für eine Zeit, die ausreicht, um die Keime zu markieren. Anschließend wird die überschüssige Lösung oder Dispersion des Fluoreszenzmarkers durch erneutes Anlegen eines Über- oder Unterdrucks über den porösen Träger entfernt. Schließlich kann gegebenenfalls zur Entfernung überschüssigen Fluoreszenzmarkers die Probe einer einfachen oder mehrfachen Wäsche mit Wasser, Pufferlösungen oder anderen Flüssigkeiten unterzogen werden. Nach Abziehen des Wassers über den porösen Träger durch Anlegen von Über- oder Unterdruck kann der poröse Träger dann schließlich vom Probengefäß abgetrennt werden, so daß ein mit fluoreszenzmarkierten Keimen belegter poröser Träger, insbesondere Membranfilter oder Siliciummikrosieb, resultiert. Dieser kann unmittelbar, d. h. im allgemeinen ohne weitere Aufbereitung der Probe oder Behandlung der Probe bzw. des Filters, der Messung bzw. Detektion zugeführt werden. Im Rahmen der Messung wird der mit fluoreszenzmarkierten Keimen belegte Träger dann mit Licht geeigneter Wellenlänge bestrahlt und hierbei sozusagen abgerastert bzw. abgescannt. Der ermittelte Meßwert korreliert mit der Keimzahl auf dem Membranfilter bzw. in der Probe.

Der der vorliegenden Erfindung zugrundeliegende Meßaufbau beinhaltet einige "Abweichungen" von einem üblichen Fluoreszenzmikroskop und geht an manchen Stellen einen Schritt weiter.

Der Detektor kann beispielsweise die folgenden Komponenten umfassen: Einen Probenhaltertisch, der in alle drei Raumrichtungen verfahrbar ist. Die Schrittweise seiner Positionierung liegt z. B. bei etwa 2 µm. Die Steuerung des Tisches erfolgt beispielsweise rechnergestützt, wobei in einem entsprechenden Meßprogramm die Parameter zum Abrastern eines Trägers bzw. Membranfilters oder Siliciummikrosiebs hinterlegt sind. Diese Parameter beschreiben eine automatische Schärfeeinstellung, die Rasterbreite in x- und y-Richtung, das Tiefenprofil in z-Richtung sowie die Fläche der zu untersuchenden Probe. Der Probenhaltertisch selbst kann z. B. derart geformt sein, daß in bevorzugter Weise Standardobjektträger mit den Maßen 26 mm Breite und 76 mm Länge (ISO Norm 8037/l) fest fixiert werden können.

Die Probe wird z. B. mit einer LED (light emitting diode) bestrahlt. Die Verwendung einer LED hat den Vorteil, daß das zu untersuchende Präparat einer nur geringen thermischen Belastung ausgesetzt und somit länger haltbar ist. Die Anregungswellenlänge der LED richtet sich insbesondere nach dem Absorptionsmaximum des verwendeten Fluoreszenzfarbstoffs. Das Licht der LED wird über einen dichroitischen Strahlenteiler auf die Probe gelenkt. Die von den Keimen emittierte Fluoreszenzstrahlung kann auf ihrem Weg durch ein Mikroskopobjektiv zu einer CCD-Kamera spektral gefiltert werden. Dadurch kann die Fluoreszenzstrahlung wellenlängenabhängig detektiert werden; man erhält gewissermaßen einen "spektralen Fingerabdruck" der Keime.

Mit Hilfe einer Autofokusfunktion kann eine automatische Schärfeeinstellung in verschiedenen, bestimmten Ebenen erfolgen. Die so erhaltenen Bildausschnitte haben beispielsweise unter bevorzugter Benutzung eines Mikroskopobjektivs mit 10facher Vergrößerung eine Größe von ca. 1,5 mm x 1,0 mm. Der Autofokus dient zur Festlegung einer bestimmten Bildebene. Von dieser aus wird in definierten Verfahrensschritten eine Bewegung des zu untersuchenden Präparates in z-Richtung (also vertikal zur Fläche des Präparates) durchgeführt. Auf diese Weise werden Bilder in verschiedenen Tiefenebenen aufgenommen. Eine anschließende Projektion der Bildebenen liefert schließlich ein tiefenscharfes Gesamtbild.

Bei der Gesamtanalyse eines Trägers bzw. Membranfilters oder Siliciummikrosiebs liefern so mehrere Gesichtsfelder (z. B. in der bevorzugten Größe 1,5 mm x 1,0 mm) in verschiedenen Tiefen unter Verwendung unterschiedlicher optischer Filter eine relativ große Menge an Daten. Ferner können in einem bestimmten Gesichtsfeld bei bestehender Tiefe und optischem Filter bei einer längeren Belichtungszeit eine Reihe von Bildsequenzen aufgenommen werden.

Die so erzeugten Bilder werden unter Berücksichtigung verschiedener Auswerteparameter auf ihren tatsächlichen Keimgehalt hin beurteilt. Zum einen spielt die Größe und die Form eines detektierten Partikels eine wichtige Rolle. Da die nachzuweisenden Keime im allgemeinen kleiner als 6 µm sind, können alle größeren Teilchen vernachlässigt werden. In diesem Zusammenhang ist auch die Form zu sehen; denn bei der hier genannten, bevorzugten Vergrößerung ist das Verhältnis der Hauptachsen der Keime in erster Näherung 1 : 1, d. h. die Keime erscheinen (mit Ausnahme der Hyphenform eines Pilzes) als runde Gebilde. Partikel, die ein anderes Hauptachsenverhältnis aufweisen, werden somit ebenfalls außer acht gelassen.

Als weiterer Parameter kommt die Analyse der Fluoreszenzcharakteristik hinzu. Hierbei ist nicht nur die Intensität der Strahlung bei einer bestimmten Wellenlänge oder einem Wellenlängenbereich von Interesse. Vielmehr ist das Verhältnis der Fluoreszenzintensitäten bei verschiedenen Wellenlängen/Wellenlängenbereichen zur Diskriminierung zwischen Keimen und anderen Partikeln entscheidend. Diese Wellenlängenunterschiede werden in dem zuvor genannten tiefenscharfen Bild als Falschfarben zusätzlich dargestellt. Zur Abtrennung des Anregungslichtes wird ein dichroitischer Strahlenteiler mit einer "Kante" bei 500 nm eingesetzt. Dadurch wird erreicht, daß das Anregungslicht an dem Filter fast vollständig reflektiert wird, während das von den angefärbten lebenden Keimen emittierte Fluoreszenzlicht ab 520 nm Wellenlänge fast komplett durchgelassen wird. Im Fluoreszenzstrahlengang ist ein Langpaßfilter mit einer "Kante" bei 520 nm angeordnet, um das intensive Anregungslicht der LED noch weiter abzuschwächen. Alle weiteren Filter sind in ein Filterrad integriert, wobei eine Position ("Position 1 ") des Filterrads ohne Filter bleibt; dieses dient zur Messung des insgesamt von der Probe abgestrahlten Fluoreszenzlichts. Auf zwei weiteren Positionen ("Positionen 2 und 3") sind Bandpaßfilter eingesetzt, die mit unterschiedlichen Breiten das grüne Fluoreszenzlicht herausfiltern. Drei weitere Filter an den verbleibenden Positionen des Filterrads ("Positionen 4 bis 6") sind Langpaßfilter mit immer weiter in den roten Bereich verschobenen "Absorptionskanten".

Letztens wird die bei einer bestimmten Bestrahlungszeit ermittelte Fluoreszenzintensität ausgewertet. Es handelt sich hierbei um keine Messung der Fluoreszenzlebensdauer, bei der unter einmaliger Anregung die Zeitdauer des "Nachleuchtens" detektiert wird. Bei der vorliegenden Erfindung wird vielmehr ein Gesichtsfeld für eine bestimmte Zeit belichtet, eine Aufnahme des Feldes durchgeführt, erneut belichtet usw. Dieses, mit der LED erzeugte Ausbleichen ("Bleaching") dient in entscheidender Weise zur Differenzierung von Keimen und anderen, für die Bestimmung der Keimzahl unerheblicher Teilchen ("Störpartikeln"). Da Keime nur eine endliche Menge an Fluoreszenzfarbstoff aufnehmen, ist dieser nach einer bestimmten Zeit durch die Einstrahlung der LED gebleicht; die Keime leuchten nur noch schwach oder überhaupt nicht mehr. Partikel hingegen, die nicht vom Fluoreszenzfarbstoff angefärbt wurden und aufgrund intrinsischer Eigenschaften eine Fluoreszenz hervorrufen (z. B. Kunststoffpartikel), bleiben in ihrer Intensität von der Bestrahlungszeit nahezu unbeeinflußt.

Das eingesetzte System ist in der Lage, vollständig automatisiert den Fokus eines betrachtenden Gesichtsfelds zu finden. Zur möglichst genauen Abbildung der auf den Membranfiltern vorhandenen Keime und anderen Partikeln erfolgt eine Autofokuseinstellung nach jedem Verfahrensschritt bzw. in jedem Gesichtsfeld erneut.

Bei längerer Belichtungszeit besteht ein fundamentaler Unterschied in der Fluoreszenzintensität zwischen mit Fluoreszenzfarbstoffen angefärbten Keimen und Störpartikeln, d. h. Teilchen, die eine Eigenfluoreszenz aufweisen. Im Fall der Keime wird der Farbstoff durch das energiereiche Licht allmählich ausgebleicht. Bei aufgrund ihrer intrinsischen Eigenschaften fluoreszierenden Teilchen (Eigenfluoreszenz) ruft dagegen eine längere Belichtung keine oder nur eine sehr schwache Änderung der Intensität hervor. Dieses Kriterium ist sehr entscheidend, wenn gleich große und in ihrer spektralen Charakteristik ähnliche Teilchen voneinander unterschieden werden sollen. Es ist in diesem Zusammenhang zu beachten, daß dieses beschriebene Ausbleichverfahren zur Differenzierung nicht mit den Fluoreszenzlebensdaueruntersuchungen (vgl. z. B. H. J. Tanke et al., loc. cit.) gleichgesetzt werden kann.

Die Kombination der Charakteristiken Form/Größe und spektrale Eigenschaften sowie Ausbleichverhalten von Teilchen - sowohl Keime als auch "Störpartikel" - führt zu einer extrem hohen Sicherheit bei der Differenzierung bzw. Diskriminierung. Somit ist eine nachfolgende mikroskopische Inspektion durch den Bediener nicht mehr erforderlich. Auf diese Art und Weise ist es möglich, automatische Analysen im Bereich < 10 KBE / ml reproduzierbar und verläßlich durchzuführen.

Mit dem erfindungsgemäßen Verfahren sind eine Vielzahl von Vorteile verbunden, von denen die wesentlichen, jedoch nicht beschränkend, im folgenden aufgeführt sind:

Ein Vorteil des erfindungsgemäßen Verfahrens ist darin zu sehen, daß es automatisiert durchgeführt werden kann. Durch die vollständige Automatisierung des gesamten Ablaufs läßt sich das Verfahren einfacher, schneller und reproduzierbarer durchführen. Dies bringt Vorteile hinsichtlich Kosten, Personalaufwand und Empfindlichkeit mit sich. Die hohe Reproduzierbarkeit bei der Durchführung des Untersuchungen ist ebenfalls von großem Vorteil.

Ein weiterer Vorteil des erfindungsgemäßen Verfahrens besteht in der Verwendung standardisierter bzw. herkömmlicher Komponenten: Das für die Durchführung des erfindungsgemäßen Verfahrens erforderliche Gesamtsystem ist in der Weise integriert, daß eine Vielzahl standardisierter bzw. herkömmlicher Komponenten (Gefäße, Medien, Filter etc.) verwendet werden kann und damit der Bedieneraufwand reduziert und die Sicherheit des Verfahrens erhöht wird.

Ein anderer Vorteil des erfindungsgemäßen Verfahrens besteht in der einfachen Detektion und Auswertung: Das erfindungsgemäße Verfahren kann beispielsweise in einem geeigneten Probengefäß durchgeführt werden, so daß die markierten Keime auf einem geeigneten Träger, z. B. einer Filtermembran, präpariert werden.

Wiederum ein weiterer Vorteil des erfindungsgemäßen Verfahrens besteht in der Geschwindigkeit der Durchführung des erfindungsgemäßen Verfahrens: Das erfindungsgemäße Verfahren erlaubt eine Bestimmung der Keimzahl bereits nach einer Zeit zwischen wenigen Minuten, abhängig von der Art der Keime und ihrer Anzahl. Konventionelle Kulturmethoden benötigen dagegen bis zu mehreren Tage.

Auch die hohe Empfindlichkeit des erfindungsgemäßen Verfahrens ist von besonderem Vorteil: Das erfindungsgemäße Verfahren erlaubt die Bestimmung von Keimen auch in großer Verdünnung. Beschleunigte Kulturverfahren sind nicht ausreichend empfindlich für eine Nachweisgrenze von 10 KBE pro Milliliter Probevolumen.

Schließlich muß ein weiterer Vorteil des erfindungsgemäßen Verfahrens in der Bedienerfreundlichkeit gesehen werden: Der gesamte Prozeß der Bestimmung der Keimbelastung einer Probe besteht bei automatisierter Durchführung lediglich aus dem Einfüllen der Probe und dem Starten des Ablaufs. Im Anschluß erhält der Benutzer den numerischen Wert für die Keimbelastung. Der Aufwand für Probenaufbereitung und Messung ist minimal. Das System läßt sich damit auch in idealer Weise in Prozeßsysteme zur Qualitätsüberwachung, -sicherung und -dokumentation einbinden.

Die vorliegende Erfindung kann mit einer Vorrichtung zur quantitativen und/oder qualitativen Bestimmung von Keimen in einer Probe mittels Fluoreszenzmarkierung bzw. mittels eines Fluoreszenzmarkers durchgeführt werden wobei die Vorrichtung derart ausgebildet ist, daß der zeitabhängige Verlauf der Fluoreszenzemission zur Diskriminierung zwischen auf die fluoreszenzmarkierten Keime zurückgehenden Meßsignalen einerseits und etwaigen Störsignalen andererseits erfaßbar ist.

Insbesondere betrifft dies eine Vorrichtung zur quantitativen und/oder qualitativen Bestimmung von Keimen in einer Probe nach einem Verfahren mit Probenaufbereitung und anschließender Detektion und/oder Auswertung, wobei mittels der Vorrichtung im Zuge der Probenaufbereitung eine Markierung zumindest eines Teils der in der Probe vorhandenen Keime mittels mindestens eines Fluoreszenzmarkers durchführbar ist und die Detektion und/oder Auswertung unter Nutzung des Fluoreszenzmarkers durch Erfassung und/oder Messung der Fluoreszenzemission durchführbar ist, insbesondere zur Durchführung des zuvor beschriebenen erfindungsgemäßen Verfahrens, wobei die Vorrichtung
- einen Probenaufnahmebehälter,
- einen an einem Auslaß des Probenaufnahmebehälters, insbesondere an dessen Boden, angeordneten, vorzugsweise porösen Träger zur Aufbringung und/oder Fixierung der Keime, insbesondere Membranfilter oder Siliciummikrosieb, wobei der Träger derart ausgebildet ist, daß er die zu detektierenden Keime zurückhält und/oder in bezug auf die zu detektierenden Keime undurchlässig ist, und
- ein Detektionssystem, das zur Ausführung einer Fluoreszenzemissionsmessung ausgestaltet ist und an dem der Probenaufnahmebehälter mit dem Träger und/oder der aus dem Probenaufnahmebehälter entnommene Träger zu Zwecken der Detektion und/oder Auswertung positionierbar ist, wobei das Detektionssystem eine vorzugsweise computergesteuerte Steuer- und/oder Auswerteeinrichtung als Teil des Detektionssystems und/oder zur Steuerung des Detektionssystems aufweist und eine Strahlungsquelle umfaßt, mittels derer die fluoreszenzmarkierten Keime für eine definierte Zeitdauer einer Anregungsstrahlung einer definierten Wellenlänge oder eines definierten Wellenlängenbereichs ausgesetzt werden können, wobei das Detektionssystem, insbesondere die Steuer- und/oder Auswerteeinrichtung, Mittel zur Erfassung des zeitabhängigen Verlaufs der infolge der Anregung erzeugten Fluoreszenzemissionsstrahlung aufweist, so daß eine Diskriminierung zwischen auf die fluoreszenzmarkierten Keime zurückgehenden Meßsignalen einerseits und etwaigen Störsignalen andererseits erfaßbar ist,
aufweist.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

## Patentansprüche

1. Verfahren zur quantitativen und/oder qualitativen Bestimmung von Keimen in einer Probe, wobei das Verfahren einen Verfahrenschritt (a) der Probenaufbereitung und einen Verfahrensschritt (b) der Detektion und/oder Auswertung umfaßt, wobei in Verfahrensschritt (a) eine Markierung zumindest eines Teils der in der Probe vorhandenen Keime mittels mindestens eines Fluoreszenzmarkers erfolgt und in Verfahrensschritt (b) eine quantitative und/oder qualitative Detektion und/oder Auswertung erfolgt, wobei die in Verfahrensschritt (b) durchgeführte Detektion und/oder Auswertung durch Erfassung und/oder Messung der Fluoreszenzemission erfolgt, wobei in Verfahrensschritt (b) die in Verfahrensschritt (a) aufbereitete Probe mit den fluoreszenzmarkierten Keimen für eine definierte Zeitdauer einer Anregungsstrahlung einer definierten Wellenlänge oder eines definierten Wellenlängenbereichs ausgesetzt und der zeitabhängige Verlauf der infolge der Anregung erzeugten Fluoreszenzemissionsstrahlung erfaßt wird, **dadurch gekennzeichnet, daß** die Kinetik des Abbaus und/oder Ausbleichens des Fluoreszenzmarkers erfaßt wird, so daß eine Diskriminierung zwischen auf die fluoreszenzmarkierten Keime zurückgehenden Meßsignalen einerseits und etwaigen Störsignalen andererseits ermöglicht wird und auf diese Weise eine quantitative und/oder qualitative Bestimmung der fluoreszenzmarkierten Keime in der Probe erfolgt, **dadurch gekennzeichnet, dass** zusätzlich eine Diskriminierung über die Größe und/oder Form der emittierenden Partikel erfolgt.

2. Verfahren nach Anspruch 1, **dadurch gekennzeichnet, daß** die Erfassung der Kinetik über die Erfassung des zeitabhängigen Verlaufs der Intensität der Fluoreszenzemissionssignale erfolgt.

3. Verfahren nach einem oder mehreren der vorangehenden Ansprüche, **dadurch gekennzeichnet, daß** auf Basis der durch Fluoreszenzemission ermittelten Meßwerte die in der Probe vorhandene Zahl an Keimen bestimmt werden kann, insbesondere mittels geeigneter Kalibrierung.

4. Verfahren nach einem oder mehreren der vorangehenden Ansprüche, **dadurch gekennzeichnet, daß** in zwei oder mehreren, zeitlich aufeinanderfolgenden Zeitintervallen mit Anregungsstrahlungen von jeweils voneinander unterschiedlichen, aber jeweils definierten Wellenlängen oder Wellenlängenbereichen auf die aufbereitete Probe mit den fluoreszenzmarkierten Keimen eingestrahlt wird und/oder daß in mindestens zwei zeitlich aufeinanderfolgenden (Zeit-)Intervallen die Fluoreszenzstrahlung bei jeweils voneinander unterschiedlichen, aber jeweils definierten Wellenlängen oder Wellenlängenbereichen von der aufbereiteten Probe mit den fluoreszenzmarkierten Keimen detektiert wird.

5. Verfahren nach einem oder mehreren der vorangehenden Ansprüche, **dadurch gekennzeichnet, daß** die Wellenlänge oder der Wellenlängenbereich der Anregungsstrahlung auf die Fluoreszenzcharakteristik des Fluoreszenzmarkers und/oder der fluoreszenzmarkierten Keime abgestimmt ist, insbesondere auf deren Absorptionsmaxima in bezug auf die Fluoreszenzemission.

6. Verfahren nach einem oder mehreren der vorangehenden Ansprüche, **dadurch gekennzeichnet, daß** zusätzlich eine Diskriminierung über die Analyse der Fluoreszenzcharakteristik erfolgt, insbesondere wobei das Verhältnis der Fluoreszenzintensitäten bei verschiedenen, aber definierten Wellenlängen oder Wellenlängenbereichen erfaßt wird.

7. Verfahren nach einem oder mehreren der vorangehenden Ansprüche, **dadurch gekennzeichnet, daß** die Keime auf einem Träger aufgebracht und/oder fixiert sind.

8. Verfahren nach einem oder mehreren der vorangehenden Ansprüche, **dadurch gekennzeichnet, daß** der Träger porös ausgebildet ist, insbesondere wobei die Poren des Trägers derart gewählt sind, daß die Porengröße kleiner als die in der Probe vorhandenen Keime ist, und/oder daß der Träger derart ausgebildet ist, daß er die Keime zurückhält und/oder in bezug auf die Keime undurchlässig ist.

9. Verfahren nach einem oder mehreren der vorangehenden Ansprüche, **dadurch gekennzeichnet, daß** die fluoreszenzmarkierten Keime bei der in Verfahrensschritt (a) durchgeführten Probenaufbereitung auf einem insbesondere porösen Membranfilter, vorzugsweise einem Polycarbonatmembranfilter, oder einem Siliciummikrosieb aufgebracht werden, wobei das Membranfilter oder das Siliciummikrosieb so ausgebildet ist, daß es die Keime zurückhält und/oder in bezug auf die Keime undurchlässig ist.

10. Verfahren nach Anspruch 9, **dadurch gekennzeichnet, daß** die Größe der Poren des Membranfilters oder des Siliciummikrosiebs so gewählt ist, daß die Porengröße kleiner als die in der Probe vorhandenen Keime ist.

11. Verfahren nach einem der Ansprüche 7 bis 10, **dadurch gekennzeichnet, daß** die Detektion und/oder Auswertung, insbesondere ohne weitere Probenbehandlung oder -aufbereitung oder -übertragung, unmittelbar auf dem Träger, insbesondere Membranfilter oder Siliciummikrosieb, erfolgt.

12. Verfahren nach einem der Ansprüche 7 bis 11, **dadurch gekennzeichnet, daß** der Fluoreszenzmarker derart ausgewählt wird, daß er porengängig, insbesondere membrangängig oder mikrosiebgängig, in bezug auf den bei der in Verfahrensschritt (a) durchgeführten Probenaufbereitung verwendeten Träger, insbesondere Membranfilter oder Siliciummikrosieb, ist.

13. Verfahren nach einem der vorangehenden Ansprüche, **dadurch gekennzeichnet, daß** der Verfahrensschritt (a) der Probenaufbereitung auch eine Inaktivierung und/oder Entfernung von gegebenenfalls in der Probe vorhandenen keimhemmenden und/oder keimtötenden Substanzen oder Bestandteilen, insbesondere Konservierungsstoffen oder Tensiden, umfaßt, insbesondere wobei die Inaktivierung und/oder Entfernung der gegebenenfalls in der Probe vorhandenen keimhemmenden und/oder keimtötenden Substanzen oder Bestandteile **dadurch** erfolgen kann, daß die Probe mit einer geeigneten Inaktivierungs- und/oder Konditionierlösung in Kontakt gebracht wird.

14. Verfahren nach einem der vorangehenden Ansprüche, **dadurch gekennzeichnet, daß** der Fluoreszenzmarker derart ausgewählt wird, daß er mit den Keimen in Wechselwirkung tritt, insbesondere an die Keime, insbesondere an deren Zellwand (Hülle) und/oder Nukleinsäure, anbindet und/oder von den Keimen aufgenommen, insbesondere metabolisiert und/oder enzymatisch umgesetzt wird.

15. Verfahren nach einem der Ansprüche 1 bis 14, **dadurch gekennzeichnet, daß** als Fluoreszenzmarker ein keimunspezifischer Fluoreszenzmarker oder ein Gemisch keimunspezifischer Fluoreszenzmarker eingesetzt wird.

16. Verfahren nach einem der Ansprüche 1 bis 14, **dadurch gekennzeichnet, daß** als Fluoreszenzmarker ein keimspezifischer Fluoreszenzmarker oder ein Gemisch verschieden keimspezifischer Fluoreszenzmarker eingesetzt wird.

17. Verfahren nach einem der Ansprüche 1 bis 14, **dadurch gekennzeichnet, daß** als Fluoreszenzmarker ein Gemisch keimunspezifischer und keimspezifischer Fluoreszenzmarker eingesetzt wird.

18. Verfahren nach einem der Ansprüche 1 bis 17, **dadurch gekennzeichnet, daß** als Fluoreszenzmarker ein mit lebenden Keimen in Wechselwirkung tretender Fluoreszenzmarker eingesetzt wird.

19. Verfahren nach einem der Ansprüche 1 bis 17, **dadurch gekennzeichnet, daß** als Fluoreszenzmarker ein mit toten Keimen in Wechselwirkung tretender Fluoreszenzmarker eingesetzt wird.

20. Verfahren nach einem der Ansprüche 1 bis 17, **dadurch gekennzeichnet, daß** als Fluoreszenzmarker ein Gemisch von mit lebenden Keimen in Wechselwirkung tretenden Fluoreszenzmarkern und mit toten Keimen in Wechselwirkung tretenden Fluoreszenzmarkern eingesetzt wird, so daß eine lebend/tot-Differenzierung der in der Probe vorhandenen Keime erfolgt.

21. Verfahren nach einem der vorangehenden Ansprüche, **dadurch gekennzeichnet, daß** als Fluoreszenzmarker ein in der Epifluoreszenzmikroskopie oder in der DEFT (Direkte Epifluoreszenz-Filter-Technik) oder in der MMCF (Membranfilter-Mikrokolonie-Fluoreszenz-Methode) üblicherweise zur Keimmarkierung verwendeter Fluoreszenzmarker eingesetzt wird.

22. Verfahren nach einem der vorangehenden Ansprüche, **dadurch gekennzeichnet, daß** als Fluoreszenzmarker ein Fluoreszenzfarbstoff oder aber eine Vorstufe eines solchen Fluoreszenzfarbstoffs, aus dem durch Wechselwirkung mit den Keimen, insbesondere durch Metabolisierung und/oder enzymatische Umsetzung, der Fluoreszenzfarbstoff generiert wird, eingesetzt wird.

23. Verfahren nach Anspruch 22, **dadurch gekennzeichnet, daß** der Fluoreszenzfarbstoff ausgewählt ist aus der Gruppe von 3,6-Bis[dimethylamino]acridin (Acridinorange), 4',6-Diamido-2-phenylindol (DAPI), 3,8-Diamino-5-ethyl-6-phenyl-phenanthridiniumbromid (Ethidiumbromid), 3,8-Diamino-5-[3-(diethylmethylammonio)propyl]-6-phenyl-phenanthridiniumdiiodid (Propidiumiodid), Rhodaminen wie Rhodamin B und Sulforhodamin B sowie Fluoresceinisothiocyanat.

24. Verfahren nach einem der vorangehenden Ansprüche, **dadurch gekennzeichnet, daß** als Fluoreszenzmarker eine insbesondere keimspezifische Nukleinsäuresonde eingesetzt wird, die ihrerseits fluoreszenzmarkiert ist, insbesondere mit einer fluoreszierenden Gruppe oder einem fluoreszierenden Molekül, insbesondere wobei die fluoreszierende Gruppe oder das fluoreszierende Molekül kovalent oder anderweitig an die Nukleinsäuresonde gebunden sein kann und/oder insbesondere wobei die Nukleinsäuresonde ein fluoreszenzmarkiertes Oligo- oder Polynukleotid umfaßt und/oder insbesondere wobei die Nukleinsäuresonde eine fluoreszenzmarkierte DNA- oder RNA-Sonde ist.

25. Verfahren nach Anspruch 24, **dadurch gekennzeichnet, daß** als Nukleinsäuresonde eine in der Fluoreszenz-in-situ-Hybridisierung (FISH) üblicherweise zur Markierung verwendete Nukleinsäuresonde eingesetzt wird.

26. Verfahren nach einem der vorangehenden Ansprüche, **dadurch gekennzeichnet, daß** als Fluoreszenzmarker ein insbesondere keimspezifischer Antikörper eingesetzt wird, der seinerseits fluoreszenzmarkiert ist, insbesondere mit einer fluoreszierenden Gruppe oder einem fluoreszierenden Molekül, insbesondere wobei die fluoreszierende Gruppe oder das fluoreszierende Molekül kovalent oder anderweitig an den Antikörper gebunden sein kann.

27. Verfahren nach einem der vorangehenden Ansprüche, **dadurch gekennzeichnet, daß** die Nachweisegrenze in bezug auf die Keime ≤ 100 Koloniebildende Einheiten (KBE) pro Milliliter Probevolumen, vorzugsweise ≤ 10 Koloniebildende Einheiten (KBE) pro Milliliter Probevolumen, ist.

28. Verfahren nach einem der vorangehenden Ansprüche, **dadurch gekennzeichnet, daß** es sich bei den zu bestimmenden Keimen um pathogene Keime aller Art handelt, insbesondere Mikroorganismen aller Art, insbesondere einzellige Mikroorganismen, wie Bakterien und Pilze (z. B. Hefen oder Schimmelpilze).

29. Verfahren nach einem der vorangehenden Ansprüche zur quantitativen und/oder qualitativen Bestimmung von Keimen in Lebensmitteln, tensidhaltigen Produkten wie Wasch- und Reinigungsmitteln, Mitteln zur Oberflächenbehandlung, Dispersionsprodukten, Kosmetika, Hygieneprodukten und Produkten der Körperpflege, Pharmazeutika, Klebstoffen, Kühlschmierstoffen, Lacken und (Lack-)Koagulationen sowie Rohstoffen und Ausgangsstoffen für die vorgenannten Produkte.

30. Verfahren nach einem der vorangehenden Ansprüche zur quantitativen und/oder qualitativen Bestimmung von Keimen in filtrierbaren, insbesondere flüssigen und/oder fließfähigen Produkten.

31. Verfahren nach einem der vorangehenden Ansprüche, **dadurch gekennzeichnet, daß** das Verfahren automatisiert durchgeführt wird.

32. Verfahren nach einem der vorangehenden Ansprüche, **dadurch gekennzeichnet, daß** das Verfahren im Rahmen der Produktions- und/oder Qualitätskontrolle durchgeführt wird.

## Claims

1. A method for quantitatively and/or qualitatively identifying germs in a sample, wherein the method comprises a process step (a) for sample preparation and a process step (b) for detection and/or evaluation, wherein in process step (a) labeling of at least one portion of the germs present in the sample is performed by means of at least one fluorescent marker and in process step (b) quantitative and/or qualitative detection and/or evaluation is performed, wherein the detection and/or evaluation carried out in process step (b) is performed by recording and/or measuring fluorescence emission, wherein in process step (b) the sample prepared in process step (a) with the fluorescently labeled germs is exposed to an excitation radiation of a defined wavelength or a defined wavelength range for a defined period of time and the time-dependent course of the fluorescence emission radiation produced as a result of the excitation is recorded, **characterized in that** the kinetics of the degradation and/or bleaching of the fluorescent marker are recorded, so that a discrimination is made possible between the measured signals caused by the fluorescently marked germs on the one hand and possible interfering signals on the other hand, and in this way quantitative and/or qualitative identification of the fluorescently labeled germs in the sample is performed, **characterized in that** additionally discrimination is carried out by way of size and/or shape of the emitting particles.

2. The method according to claim 1, **characterized in that** the recording of the kinetics is performed by recording the time-dependent course of the intensity of the fluorescence emission signals.

3. The method according to one or more of the preceding claims, **characterized in that** the number of germs present in the sample may be determined on the basis of the measured values determined by fluorescence emission, in particular by means of suitable calibration.

4. The method according to one or more of the preceding claims, **characterized in that** in two or more successive time intervals, excitation radiations of wavelength or wavelength ranges which differ from one another in each case but are defined in each case, are radiated on the prepared sample with the fluorescently labeled germs and/or that in at least two successive time intervals, the fluorescence radiation at wavelengths or wavelength ranges which differ from one another in each case but are defined in each case, is recorded from the prepared sample with the fluorescently labeled germs.

5. The method according to one or more of the preceding claims, **characterized in that** the wavelength or the wavelength range of the excitation radiation matches the fluorescence characteristics of the fluorescent marker and/or of the fluorescently labeled germs, in particular their absorption peaks with respect to fluorescence emission.

6. The method according to one or more of the preceding claims, **characterized in that** additionally discrimination is performed by way of analyzing the fluorescence characteristics, in particular wherein the ratio of the fluorescence intensities is recorded at different but defined wavelengths or wavelength ranges.

7. The method according to one or more of the preceding claims, **characterized in that** the germs are applied and/or fixed to a support.

8. The method according to one or more of the preceding claims, **characterized in that** the support has a porous structure, in particular wherein the pores of the support are selected in such a way that the pore size is smaller than the germs present in the sample, and/or **in that** the support is formed so as to retain the germs and/or is impermeable with respect to the germs.

9. The method according to one or more of the preceding claims, **characterized in that** the fluorescently labeled germs in the sample preparation carried in process step (a) are applied on a particularly porous membrane filter preferably a polycarbonate membrane filter, or a silicon microsieve, wherein the membrane filter or silicon microsieve is formed so that it retains the germs and/or is impermeable with respect to the germs.

10. The method according to claim 9, **characterized in that** the size of the pores of the membrane filter or of the silicon microsieve is selected so that the pore size is smaller than the germs present in the sample.

11. The method according to any of claims 7 to 10, **characterized in that** the detection and/or evaluation takes place directly on the support, in particular a membrane filter or silicone microsieve, in particular without any further sample treatment or preparation or transfer.

12. The method according to any of claims 7 to 11, **characterized in that** the fluorescence marker is selected so that it is able to pass through the pores, in particular through the membrane or the microsieve, with respect to the support, in particular a membrane filter or a silicon microsieve, used in the sample preparation carried out in process step (a).

13. The method according to any of the preceding claims, **characterized in that** process step (a) of the sample preparation also comprises inactivation and/or removal of germ-inhibiting and/or germicidal substances or ingredients possibly present in the sample, in particular preservatives or surfactants, in particular wherein the inactivation and/or removal of the germ-inhibiting and/or germicidal substances or ingredients possibly present in the sample may take place by contacting the sample with a suitable inactivating and/or conditioning solution.

14. The method according to any of the preceding claims, **characterized in that** the fluorescence marker is selected so that it interacts with the germs, in particular binds to the germs, in particular at their cell wall (envelope) and/or nucleic acid, and/or is absorbed, in particular metabolized and/or enzymatically converted, by the germs.

15. The method according to any of claims 1 to 14, **characterized in that** a germ-unspecific fluorescent marker or a mixture of germ-unspecific fluorescent markers is used as a fluorescent marker.

16. The method according to any of claims 1 to 14, **characterized in that** a germ-specific fluorescent marker or a mixture of different germ-specific fluorescent markers is used as a fluorescent marker.

17. The method according to any of claims 1 to 14, **characterized in that** a mixture of germ-unspecific and germ-specific fluorescent markers is used as a fluorescent marker.

18. The method according to any of claims 1 to 17, **characterized in that** a fluorescent marker interacting with live germs is used as fluorescent marker.

19. The method according to any of claims 1 to 17, **characterized in that** a fluorescent marker interacting with dead germs is used as a fluorescent marker.

20. The method according to any of claims 1 to 17, **characterized in that** a mixture of fluorescent markers interacting with live germs and of fluorescent markers interacting with dead germs is used as a fluorescent marker, so that a live/dead differentiation of the germs present in the sample is performed.

21. The method according to any of the preceding claims, **characterized in that** a fluorescent marker commonly used for labeling germs in epifluorescence microscopy or in DEFT (Direct Epifluorescent Filter Technique) or in MMCF (Membrane filter Micro Colony Fluorescence method) is used as a fluorescent marker.

22. The method according to any of the preceding claims, **characterized in that** a fluorescent dye or else a precursor of such a fluorescent dye is used as a fluorescent marker, from which the fluorescent dye is generated by interaction with the germs, in particular by metabolization and/or enzymatic conversion.

23. The method according to claim 22, **characterized in that** the fluorescent dye is selected from the group of 3,6-bis[dimethyl-amino)acridine (acridine orange), 4',6-diamino-2-phenylindole (DAPI), 3,8-diamino-5-ethyl-6-phenyl-phenanthridinium bromide (ethidium bromide), 3,8-diamino-5-[3-(diethylmethylammonio)propyl]-6-phenyl phenanthridinium diiodide (propidium iodide), rhodamines such as rhodamine B and sulfo-rhodamine B and fluorescein thiocyanate.

24. The method according to any of the preceding claims, **characterized in that** a particular germ-specific nucleic acid probe is used as a fluorescent marker, which itself is fluorescently labeled, in particular with a fluorescent group or a fluorescent molecule, in particular wherein the fluorescent group or the fluorescent molecule may be covalently or otherwise bound to the nucleic acid probe and/or in particular wherein the nucleic acid probe comprises a fluorescently labeled oligo- or poly-nucleotide and/or in particular wherein the nucleic acid probe is a fluorescently labeled DNA or RNA probe.

25. The method according to claim 24, **characterized in that** a nucleic acid probe currently used for labeling in Fluorescence In Situ Hybridization (FISH) is used as a nucleic acid probe.

26. The method according to any of the preceding claims, **characterized in that** a particular germ-specific antibody is used as a fluorescent marker, which itself is fluorescently labeled, in particular with a fluorescent group or a fluorescent molecule, in particular wherein the fluorescent group or the fluorescent molecule may be bound to the antibody either covalently or otherwise.

27. The method according to any of the preceding claims, **characterized in that** the detection limit with respect to the germs is ≤ 100 Colony Forming Units (CFU) per milliliter of sample volume, preferably ≤ 10 s Colony Forming Units (CFU) per milliliter of sample volume.

28. The method according to any of the preceding claims, **characterized in that** as for the germs to be identified, these are pathogenic germs of any kind, in particular microorganisms of any kind, in particular unicellular microorganisms such as bacteria and fungi (for example yeasts or mold fungi).

29. The method according to any of the preceding claims for quantitatively and/or qualitatively identifying germs in food products, surfactant-containing products such as detergents and cleaners, surface treatment agents, dispersion products, cosmetics, hygiene products and body care products, pharmaceuticals, adhesives, cooling lubricants, paints and (paint)-coagulations and also raw materials and starting materials for the aforementioned products.

30. The method according to any of the preceding claims, for quantitatively and/or qualitatively identifying germs in filterable, in particular liquid and/or free-flowing, products.

31. The method according to any of the preceding claims, **characterized in that** the method is carried out in an automated way.

32. The method according to any of the preceding claims, **characterized in that** the method is carried out within the framework of production control and/or quality control.

## Revendications

1. Procédé pour la détermination quantitative et/ou qualitative de germes dans un échantillon, le procédé comprenant une étape opératoire (a) de préparation de l'échantillon et une étape opératoire (b) de détection et/ou d'évaluation, procédé dans lequel, à l'étape opératoire (a), a lieu un marquage d'au moins une partie des germes présents dans l'échantillon au moyen d'au moins d'un marqueur de fluorescence et, à l'étape opératoire (b), a lieu une détection et/ou une évaluation quantitative et/ou qualitative ; dans lequel la détection et/ou l'évaluation mises en oeuvre à l'étape opératoire (b) ont lieu par enregistrement et/ou par mesure de l'émission de fluorescence ; dans lequel, à l'étape opératoire (b), l'échantillon préparé à l'étape opératoire (a) comprenant des germes marqués par fluorescence est exposé pendant un laps de temps défini à une irradiation d'excitation d'une longueur d'onde définie ou d'une plage de longueurs d'ondes définie et l'allure temporelle de l'irradiation d'émission de fluorescence générée suite à l'excitation est enregistrée, **caractérisé en ce que** l'on enregistre la cinétique de la dégradation et/ou de l'extinction du marqueur de fluorescence, si bien que l'on peut procéder à une discrimination entre des signaux de mesure qui dérivent des germes marqués par fluorescence d'une part, et des signaux parasite éventuels d'autre part, et si bien que l'on obtient de cette manière une détermination quantitative et/ou qualitative des germes marqués par fluorescence dans l'échantillon, **caractérisé en ce qu'**on procède en outre à une discrimination par rapport à la dimension et/ou à la forme des particules émettrices.

2. Procédé selon la revendication 1, **caractérisé en ce que** l'enregistrement de la cinétique a lieu via l'enregistrement de l'allure temporelle de l'intensité des signaux d'émission de fluorescence.

3. Procédé selon une ou plusieurs des revendications précédentes, **caractérisé en ce que** l'on peut déterminer le nombre des germes présents dans l'échantillon sur base des valeurs de mesure déterminée par l'émission de fluorescence, en particulier au moyen d'un étalonnage approprié.

4. Procédé selon une ou plusieurs des revendications précédentes, **caractérisé en ce que**, à deux intervalles ou plus qui se succèdent dans le temps, on irradie l'échantillon préparé comprenant les germes marqués par fluorescence avec des irradiations d'excitation de longueurs d'ondes ou de plages de longueurs d'ondes qui diffèrent les unes des autres dans chaque cas, mais qui sont définies dans chaque cas, et/ou en ce qu'on détecte, dans au moins deux intervalles (de temps) qui se succèdent dans le temps le rayonnement de fluorescence, à des longueurs d'ondes ou dans des plages de longueurs d'onde qui diffèrent les unes des autres dans chaque cas, mais qui sont définies dans chaque cas, de l'échantillon préparé comprenant les germes marqués par fluorescence.

5. Procédé selon une ou plusieurs des revendications précédentes, **caractérisé en ce que** la longueur d'onde ou la plage de longueurs d'ondes du rayonnement d'excitation est assortie à la caractéristique de fluorescence du marqueur de fluorescence et/ou des germes marqués par fluorescence, en particulier à leurs maxima d'absorption par rapport à l'émission de fluorescence.

6. Procédé selon une ou plusieurs des revendications précédentes, **caractérisé en ce qu'**on procède en outre à une discrimination en ce qui concerne l'analyse de la caractéristique de fluorescence, en particulier dans lequel on enregistre le rapport des intensités de fluorescence dans le cas de longueurs d'ondes ou de plages de longueurs d'ondes différentes, mais définies.

7. Procédé selon une ou plusieurs des revendications précédentes, **caractérisé en ce que** les germes sont appliqués et/ou sont fixés sur un support.

8. Procédé selon une ou plusieurs des revendications précédentes, **caractérisé en ce que** le support est réalisé pour être poreux, en particulier dans lequel les pores du support sont sélectionnés de telle sorte que la dimension des pores est inférieure à celle des germes présents dans l'échantillon, et/ou **en ce que** le support est réalisé de telle sorte qu'il retient les germes et/ou de telle sorte qu'il est imperméable par rapport aux germes.

9. Procédé selon une ou plusieurs des revendications précédentes, **caractérisé en ce que** les germes marqués par fluorescence, lors de la préparation de l'échantillon réalisée à l'étape opératoire (a), sont appliqués sur un filtre à membrane en particulier poreux, de préférence sur un filtre à membrane de polycarbonate ou sur un microtamis en silicium, le filtre à membrane ou le microtamis en silicium étant réalisés de telle sorte qu'il retient les germes et/ou qu'il est imperméable par rapport aux germes.

10. Procédé selon la revendication 9, **caractérisé en ce que** la dimension des pores du filtre à membrane ou du microtamis en silicium est sélectionnée de telle sorte que la dimension des pores est inférieure à celle des germes présents dans l'échantillon.

11. Procédé selon l'une quelconque des revendications 7 à 10, **caractérisé en ce que** la détection et/ou l'évaluation ont lieu en particulier en l'absence d'un traitement ultérieur ou d'une préparation ultérieure ou d'un transfert ultérieur de l'échantillon, directement sur le support, en particulier sur un filtre à membrane ou sur un microtamis en silicium.

12. Procédé selon l'une quelconque des revendications 7 à 11, **caractérisé en ce que** le marqueur de fluorescence est sélectionné de telle sorte qu'il est à même de traverser des pores, en particulier à même de traverser des membranes ou à même de traverser des microtamis, par rapport au support, en particulier au filtre à membrane ou au microtamis en silicium que l'on utilise lors de la préparation de l'échantillon réalisée à l'étape opératoire (a).

13. Procédé selon une ou plusieurs des revendications précédentes, **caractérisé en ce que** l'étape opératoire (a) de la préparation de l'échantillon comprend également une inactivation et/ou une élimination de substances ou de constituants inhibant les germes et/ou germicides, en particulier des substances de conservation ou d'agents tensioactifs, qui sont présents le cas échéant dans l'échantillon, en particulier dans lequel l'inactivation et/ou l'élimination des substances ou des constituants inhibant les germes et/ou germicides présents le cas échéant dans l'échantillon peut avoir lieu de telle sorte que l'on met l'échantillon en contact avec une solution d'inactivation et/ou de conditionnement appropriée.

14. Procédé selon une ou plusieurs des revendications précédentes, **caractérisé en ce que** le marqueur de fluorescence est sélectionné de telle sorte qu'il entre en interaction avec les germes, en particulier qu'il se lie aux germes, en particulier à leur paroi cellulaire (enveloppe) et/ou à leurs acides nucléiques, et/ou **en ce qu'**il est absorbé par les germes, en particulier, **en ce qu'**il est métabolisé et/ou soumis à une mise en réaction enzymatique.

15. Procédé selon l'une quelconque des revendications 1 à 14, **caractérisé en ce qu'**on met en oeuvre, à titre de marqueur de fluorescence, un marqueur de fluorescence non spécifique à des germes ou encore un mélange de marqueurs de fluorescence non spécifiques à des germes.

16. Procédé selon l'une quelconque des revendications 1 à 14, **caractérisé en ce qu'**on met en oeuvre, à titre de marqueur de fluorescence, un marqueur de fluorescence spécifique à des germes ou encore un mélange de marqueurs de fluorescence différents spécifiques à des germes.

17. Procédé selon l'une quelconque des revendications 1 à 14, **caractérisé en ce qu'**on met en oeuvre, à titre de marqueur de fluorescence, un mélange de marqueurs de fluorescence non spécifiques à des germes et de marqueurs de fluorescence spécifiques à des germes.

18. Procédé selon l'une quelconque des revendications 1 à 17, **caractérisé en ce qu'**on met en oeuvre, à titre de marqueur de fluorescence, un marqueur de fluorescence qui entre en interaction avec des germes vivants.

19. Procédé selon l'une quelconque des revendications 1 à 17, **caractérisé en ce qu'**on met en oeuvre, à titre de marqueur de fluorescence, un marqueur de fluorescence qui entre en interaction avec des germes morts.

20. Procédé selon l'une quelconque des revendications 1 à 17, **caractérisé en ce qu'**on met en oeuvre, à titre de marqueur de fluorescence, un mélange de marqueurs de fluorescence qui entrent en interaction avec des germes vivants et de marqueurs de fluorescence qui entrent en interaction avec des germes morts, si bien que l'on obtient une différenciation vivant/mort des germes qui sont présents dans l'échantillon.

21. Procédé selon une ou plusieurs des revendications précédentes, **caractérisé en ce qu'**on met en oeuvre, à titre de marqueur de fluorescence, un marqueur de fluorescence habituellement utilisé dans la microscopie à épifluorescence ou dans la DEFT (technique de filtration directe par épifluorescence) ou dans la MMCF (procédé de fluorescence de microcolonies sur un filtre à membrane) pour le marquage de germes.

22. Procédé selon une ou plusieurs des revendications précédentes, **caractérisé en ce qu'**on met en oeuvre, à titre de marqueur de fluorescence, un colorant de fluorescence ou bien un précurseur d'un tel colorant de fluorescence, à partir duquel le colorant de fluorescence est généré, par interaction avec les germes, en particulier par métabolisation et/ou par mise en réaction enzymatique.

23. Procédé selon la revendication 22, **caractérisé en ce que** le colorant de fluorescence est choisi parmi le groupe de la 3,6-bis[diméthylamino]acridine (orange d'acridine), le 4',6-diamido-2-phénylindol (DAPI), le bromure de 3,8-diamino-5-éthyl-6-phényl-phénanthridinium (bromure d'éthidium) le diiodure de 3,8-diamino-5-[3-(diéthylméthylammonio)-propyl]-6-phényl-phénanthridinium (iodure de propidium), des rhodamines telles que la rhodamine B et la sulforhodamine B, ainsi que l'isothiocyanate de fluorescéine.

24. Procédé selon l'une quelconque des revendications précédentes, **caractérisé en ce qu'**on met en oeuvre, à titre de marqueur de fluorescence, en particulier une sonde d'acide nucléique spécifique à des germes, qui est pour sa part marquée par fluorescence, en particulier avec un groupe fluorescent ou avec une molécule fluorescente, en particulier, dans lequel le groupe fluorescent ou la molécule fluorescente peut être liée de manière covalente ou d'une autre manière à la sonde d'acide nucléique, et/ou en particulier dans lequel la sonde d'acide nucléique comprend un oligonucléotide ou un polynucléotide marqué par fluorescence, et/ou en particulier dans lequel la sonde d'acide nucléique est une sonde d'ADN ou d'ARN marquée par fluorescence.

25. Procédé selon la revendication 24, **caractérisé en ce qu'**on met en oeuvre, à titre de sonde d'acide nucléique, une sonde d'acide nucléique d'hybridation fluorescente in situ (FISH) que l'on utilise habituellement pour le marquage.

26. Procédé selon l'une quelconque des revendications précédentes, **caractérisé en ce qu'**on met en oeuvre à titre de marqueur de fluorescence, un anticorps en particulier spécifique à des germes, qui pour sa part est marqué par fluorescence, en particulier avec un groupe fluorescent ou avec une molécule fluorescente, en particulier, dans lequel le groupe fluorescent ou la molécule fluorescente peut être liée de manière covalente ou d'une autre manière à l'anticorps.

27. Procédé selon l'une quelconque des revendications précédentes, **caractérisé en ce que** la limite de décèlement, par rapport aux germes, est inférieure ou égale à 100 unités formant des colonies (UFC) par ml de volume d'échantillon, de préférence inférieure ou égale à 10 unités formant des colonies (UFC) par ml de volume d'échantillon.

28. Procédé selon l'une quelconque des revendications précédentes, **caractérisé en ce qu'**il s'agit, en ce qui concerne les germes à déterminer, de germes pathogènes de tout type, en particulier de micro-organismes de tout type, en particulier de micro-organismes unicellulaires tels que des bactéries et des champignons (par exemple des levures ou des moisissures).

29. Procédé selon l'une quelconque des revendications précédentes, pour la détermination quantitative et/ou qualitative de germes dans des aliments, des produits tensioactifs tels que des agents de lavage et de nettoyage, des agents pour le traitement des surfaces, des produits de mise en dispersion, des cosmétiques, des produits de l'hygiène et des produits pour les soins corporels, des produits pharmaceutiques, des adhésifs, des lubrifiants à froid, des laques, des vernis ou des peintures, et des coagulations (de laques, de vernis ou de peintures), ainsi que des matières premières et des substances de départ pour les produits susmentionnés.

30. Procédé selon l'une quelconque des revendications précédentes, pour la détermination quantitative et/ou qualitative de germes dans des produits filtrables, en particulier dans des produits liquides et/ou aptes à l'écoulement.

31. Procédé selon l'une quelconque des revendications précédentes, **caractérisé en ce que** le procédé est mis en oeuvre de manière automatique.

32. Procédé selon l'une quelconque des revendications précédentes, **caractérisé en ce que** le procédé est mis en oeuvre dans le cadre du contrôle de la production et/ou du contrôle de la qualité.
